# EUROPEAN PATENT APPLICATION

(11) **EP 1 955 660 A2**
(43) Date of publication of application: **13.08.2008**
(21) Application number: 08101376.5
(22) Date of filing: 07.02.2008
(51) Int. Cl.: A61B 17/00

(54) **Sprayer**

(30) Priority: 09.02.2007 JP 2007031316; 06.06.2007 JP 2007151002; 18.10.2007 JP 2007271762
(71) Applicant: Terumo Kabushiki Kaisha, Tokyo 151-0072 (JP)
(72) Inventor: Yokoyama, Kenji, Kanagawa 259-0151 (JP); Hayakawa, Koichi, Kanagawa 259-0151 (JP)
(74) Representative: Dossmann, Gérard

(57) **Abstract**

A sprayer includes first and second syringes that respectively supply first and second liquids, at least one orifice at which the first and second liquids, or a mixture of such liquids, are ejected, and first and second passages through which the first and second liquids are respectively fed toward the nozzle. At least one of the passages is provided with a deformable volume changer adapted to deform to change the internal capacity or volume. A deformer operates to deform the volume changer, and the volume of the volume changer is ultimately allowed to expand so as to have a larger volume in a state in which liquid ejection ceases as compared to during ejection.

## Description

### TECHNOLOGICAL FIELD

The present invention generally relates to a device for delivering a liquid material. More specifically, the invention pertains to a sprayer having useful application for spraying a liquid at a body region.

### BACKGROUND DISCUSSION

Sprayers have been developed in the past for mixing two or more liquids to form an anti-adhesive material or a living tissue adhesive and ejecting the mixture to a diseased region of a body.

Known sprayers like this are structured to feed ingredients, solidifiable upon mixed, e.g. a solution containing thrombin and a solution containing fibrinogen, to a diseased region or nearby in a separated state and to apply those while being mixed at the diseased region.

The existing sprayers include two syringes containing different types of liquids and passages extending in parallel therefrom so that the liquids can be ejected and mixed at the tips of the passages (i.e., at orifices). An example of such a syringe is disclosed in Japanese Patent Unexamined Publication No. 2002-282368. In order to eject the liquid, the plungers of the respective syringes are pressed.

After pressing the syringe plungers, the ejection of liquid ceases. However, because of the pressure remaining inside the passage, the liquid tends to protrude outwardly from the orifices. That is, even though the pressing of the syringe is stopped, a small amount of liquid still exits from the orifices and so the ejection of the liquid does not cease immediately In this state, liquids that are mixed together tend to solidify. As a result, clogging possibly occurs at the orifices. Even when application is to again be made by way of the clogged sprayer, the solidified portion of the liquid obstructs the liquid from ejecting at the orifices, thus raising a problem that re-application is difficult, if not impossible, to perform.

### SUMMARY OF THE INVENTION

The liquid ejecting sprayer here includes a volume changer having a capacity or volume that is adapted to be increased in the state in which the ejection of liquid ceases than in the state where liquid is being ejected. In this way, liquid is retracted toward the rear away from the orifice (s). This makes it possible to inhibit or prevent liquids from being mixed together and solidifying around the orifice(s) after ejection is completed. This inhibits or prevents clogging from occurring at and around the orifice (s) through which the liquid has been ejected. In addition, the sprayer free of clogging is capable of being used again.

The sprayer further has a gas passage. Where a deformer or deform means is provided on or associated with the gas passage to change the volume or capacity of the volume changer, the deformer can be operated by the supply of gas. Due to this, gas can be used to operate the deform means without being limited to the use of mixing two types of liquids during application. That is, gas can be utilized with effectiveness.

The sprayer can be provided with a confluence at which two types of liquids mixed together with uniformity and positiveness prior to being ejected from the nozzle. This makes it possible to suitably apply such liquids to a diseased region of a body.

The liquid transfer passage can be formed as a rigid tube provided with one or more holes, thus forming a passage-securing means that is able to reliably secure the passage of liquid through the liquid transfer passage. Even if the capacity of a liquid transfer passage decreases as a result of contraction associated with compression by the gas, the liquid transfer passage is restricted from contracting further because of the rigid tube. This positively secures the passage of liquid through the passage-forming member, thus enabling the positive application of liquids.

The sprayer here is well suited to avoiding the type of clogging difficulties associated with other known sprayers.

According to one aspect, a sprayer comprises a supplier that supplies first and second liquids of different compositions, two liquid transfer lines connected to the supplier and through which a respective one of the first and second liquids is to be supplied from the supplier, at least one orifice in fluid communication with the two liquid transfer lines, a volume changing portion on at least one of the two liquid transfer lines that is deformable to change an internal capacity of the volume changing portion, and a deformer that deforms the volume changing portion. The deformer is operable to deform the volume changing portion, whereupon a volume of the volume changing portion increases when ejection of the liquid ceases as compared to when the liquid is being ejected.

According to another aspect, a method of delivering a liquid mixture to a region of a body comprises ejecting a liquid mixture towards a region of a body by conveying first and second liquids in a forward direction along first and second liquid transfer passages respectively, mixing the liquids to produce the liquid mixture, and directing the liquid mixture at the body region. The method also involves ceasing ejection of the liquid mixture at the body region, and retracting the first liquid at a forward portion of the first liquid transfer passage in a rearward direction opposite the forward direction.

### BRIEF DESCRIPTION OF THE DRAWING FIGURES

Fig. 1 is a front perspective view of a sprayer according to a first embodiment disclosed here.
Fig. 2 is a rear perspective view of the sprayer shown in Fig. 1.
Fig. 3 is a cross-sectional view taken along the section line III-III in Fig. 1 illustrating the valve mechanism of the sprayer, in the state that the gas passage is closed.
Fig. 4 is a cross-sectional view taken along the section line III-III in Fig. 1 illustrating the valve mechanism of the sprayer, in the state that the gas passage is opened.
Fig. 5 is a somewhat schematic longitudinal cross-sectional view of the nozzle and syringe of the sprayer shown in Fig. 1, illustrating one chronological aspect of the application state.
Fig. 6 is a somewhat schematic longitudinal cross-sectional view of the nozzle and syringe of the sprayer shown in Fig. 1, illustrating one chronological aspect of the application state.
Fig. 7 is a somewhat schematic longitudinal cross-sectional view of the nozzle and syringe of the sprayer shown in Fig. 1, illustrating one chronological aspect of the application state.
Fig. 8 is a somewhat schematic longitudinal cross-sectional view of the nozzle and syringe of the sprayer shown in Fig. 1, illustrating one chronological aspect of the application state.
Fig. 9 is a somewhat schematic longitudinal cross-sectional view of the nozzle and syringe of the sprayer shown in Fig. 1, illustrating one chronological aspect of the application state.
Fig. 10 is a somewhat schematic longitudinal cross-sectional view of a nozzle and syringe of a sprayer according to a second embodiment, illustrating the state in which the liquid is being ejected.
Fig. 11 is a somewhat schematic longitudinal cross-sectional view of a nozzle and syringe of a sprayer according to the second embodiment, illustrating the state in which liquid is ceased from being ejected.
Fig. 12 is a cross-sectional view taken along the section line XII-XII in Fig. 10.
Fig. 13 is a cross-sectional view taken along the section line XIII-XII in Fig. 11.
Fig. 14 is a cross-sectional view of a nozzle of a sprayer according to a third embodiment, illustrating the state that liquid is being ejected.
Fig. 15 is a cross-sectional view of the nozzle of the sprayer according to the third embodiment, illustrating the state that liquid is ceased from being ejected.
Fig. 16 is a cross-sectional view of a nozzle of a sprayer according to fourth embodiment, illustrating the state that liquid is being ejected.
   Fig. 17 is a cross-sectional view of the nozzle of the sprayer according to the fourth embodiment, illustrating the state that liquid is ceased from being ejected.
Fig. 18 is a somewhat schematic longitudinal cross-sectional view of a nozzle and syringe of a sprayer according to a fifth embodiment.
Fig. 19 is a somewhat schematic longitudinal cross-sectional view of a nozzle and syringe of a sprayer according to a sixth embodiment, illustrating a chronological aspect of the application state.
Fig. 20 is a somewhat schematic longitudinal cross-sectional view of a nozzle and syringe of a sprayer according to a sixth embodiment, illustrating a chronological aspect of the application state.
Fig. 21 is a somewhat schematic longitudinal cross-sectional view of a nozzle and syringe of a sprayer according to a sixth embodiment, illustrating a chronological aspect of the application state.
Fig. 22 is a somewhat schematic longitudinal cross-sectional view in the vicinity of a volume changer of a sprayer according to a seventh embodiment.
Fig. 23 is a somewhat schematic longitudinal cross-sectional view of a nozzle and syringe of a sprayer according to an eighth embodiment, illustrating a chronological aspect of the application state.
Fig. 24 is a somewhat schematic longitudinal cross-sectional view of a nozzle and syringe of a sprayer according to the eighth embodiment, illustrating a chronological aspect of the application state.
Fig. 25 is a somewhat schematic longitudinal cross-sectional view of a nozzle and syringe of a sprayer according to the eighth embodiment, illustrating a chronological aspect of the application state.
Figs. 26(a) and 26(b) are somewhat schematic longitudinal cross-sectional views of a nozzle and syringe of a sprayer according to a ninth embodiment, illustrating a chronological aspect of the application state.
Figs. 27(a) and 27(b) are somewhat schematic longitudinal cross-sectional views of a nozzle and syringe of a sprayer according to the ninth embodiment, illustrating a chronological aspect of the application state.
Fig. 28 is a longitudinal cross-sectional view of a first syringe (similar also to the second syringe) to be loaded on the sprayer shown in Fig. 1.

### DETAILED DESCRIPTION

The description below describes the features, aspects and characteristics of the sprayer disclosed here according to various embodiments shown in the appended drawings.

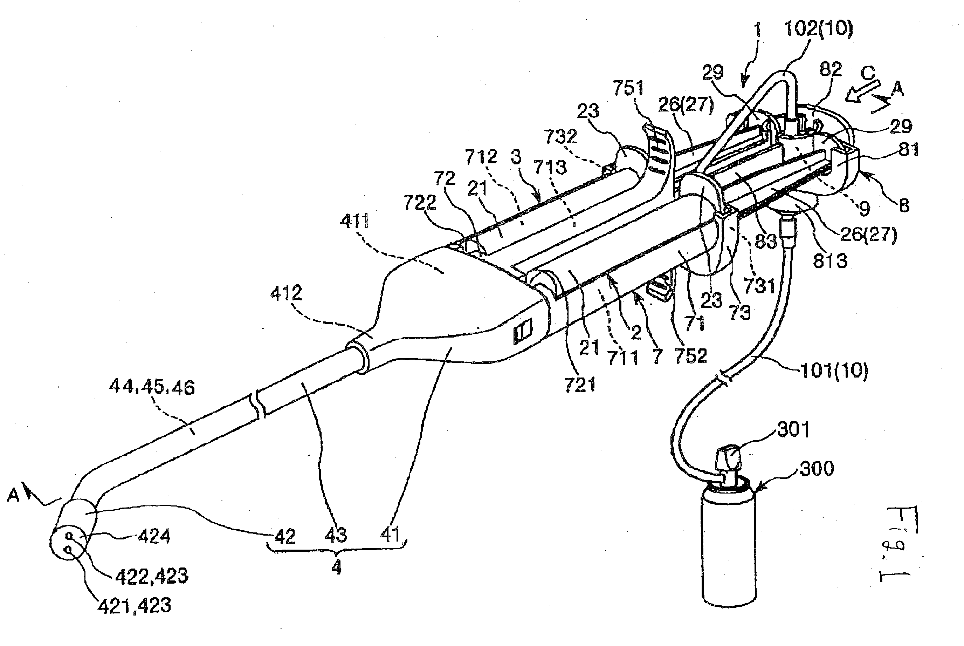

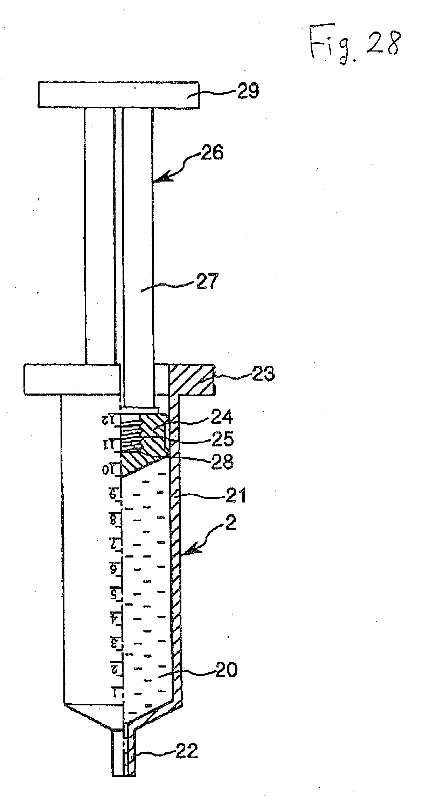

Figs. 1-9 illustrate a sprayer according to a first embodiment, while Fig. 28 is a depicts a first syringe that is to be mounted on the sprayer shown in Fig. 1. As described below in more detail, the sprayer includes two syringes, both of which can be configured in the manner shown in Fig. 28. For purposes of convenience, the left and right sides are respectively referred to as the "front" and "rear (base) " sides in Figs. 1, 2 and 5-9, while the lower and upper sides are respectively referred to as the "front" and "rear (base) " sides in Fig. 28. Meanwhile, the upper and lower sides are respectively referred to as the "upper" and "lower" sides in Figs. 1-4.

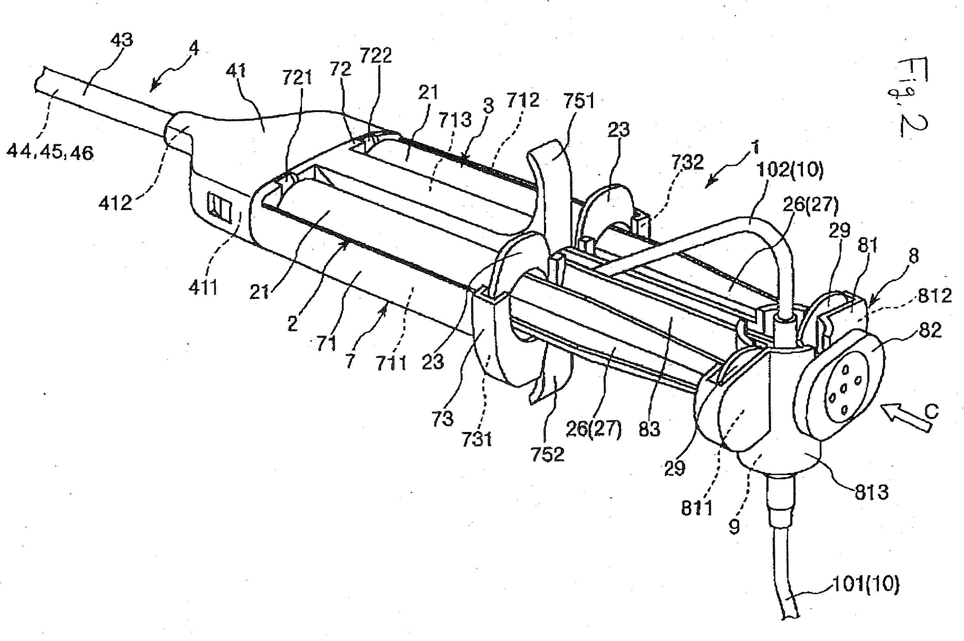

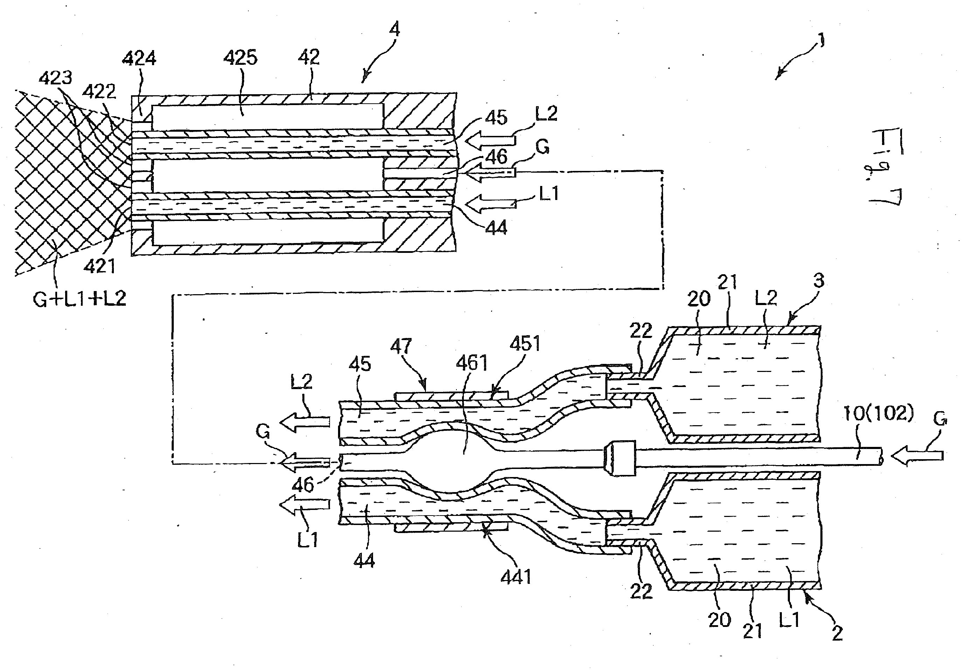

The sprayer 1 disclosed here is used with two types of liquids (first liquid L1 and second liquid L2) that are different in composition from one another and intended to be mixed together as generally shown in Fig. 7. As shown in Figs. 1 and 2, the sprayer 1 is to be used with a first syringe (supply means) 2 and a second syringe (supply means) 3 which contain the first liquid L1 and the second liquid L2, respectively.

The first syringe 2 includes an outer shell 21 and a gasket 24 that together enclose a space (reservoir space) 20. The first liquid L1 is filled in the space prior to the first syringe 2 being mounted on the sprayer 1.

As mentioned, the second liquid L2 is filled in the space 20 of the second syringe 3. The first liquid L1 to be filled in first syringe 2 and the second liquid L2 to be filled in second syringe 3 are different in their compositions or ingredients.

The first and second liquids L1, L2 are suitably selected in accordance with the application and the purpose of use of the sprayer 1, the disease case, and the like. For example, when used to administer an anti-adhesion material, one of the first and second liquids L1, L2 may contain carboxymethy dextrin modified with succinimidyl while the other may contain disodium hydrogenphosphate.

The first and second liquids L1, L2, in such a combination, are to gelatinize or form a gelatinous mixture if mixed together.

Of course, the types and combinations of first and second liquids L1, L2 are not limited to those mentioned.

The syringes each have a respective plunger 26. By pushing the respective plungers 26 of the first and second syringes 2, 3, the first and second liquids L1, L2 are supplied respectively to first and second passages 44, 45 of the nozzle 4, described in more detail below, with relative ease and reliability. The plungers 26 are to be pushed manually by the operator of the sprayer 1. Consequently, the operator can apply the liquid mixture as he/she desires.

The sprayer 1, to which is to be mounted the first and second syringes 2, 3 respectively filled with the first and second liquids L1, L2, includes a sprayer body 7, a nozzle 4, a manipulator 8, a valve means or valve mechanism 9, and a tube or gas passage 10 connected to a container (gas supply means) 300 as shown in Fig. 1.

The bombe 300 is first explained prior to describing the various parts constituting the sprayer 1. The interior of the bombe 300 is filled with a gas. In this embodiment, the interior of the bombe 300 is filed with a sterile gas G (hereinafter, referred to simply as "gas") under pressure (compressed) so that the gas G can be supplied to the sprayer 1 (nozzle 4). The bombe 300 is arranged with opening-and-closing valve (cock) 301 that opens and closes to control the supply/non-supply of gas G to the sprayer 1. When using the sprayer 1, the valve 301 is open.

As shown in Figs. 1 and 2, the sprayer body 7 is configured so that the first and second syringes 2, 3 are fixed in the sprayer in side-by-side relation. The first and second syringes 2, 3 are arranged parallel to one another. The sprayer body 7 has a base 71, a front plate (first engager) 72 provided at the front of the base 71, a rear plate (second engager) 73 provided at the rear of the base 71, and a pair of handles 751, 752 provided in the vicinity of the rear plate 73 of the base 71.

The base 71 is provided with recesses or concave regions 711, 712 that are semicircular in cross-section and arranged side-by-side in the upper portion of the base. The first syringe 2 (i.e., the outer shell 21 of the first syringe 2) is received in the recess 711 while the second syringe 3 (i.e. , the outer shell 21 of the second syringe 3) is received in the recess 712.

As mentioned, the front plate 72 is provided at the front of the base 71. The front plate 72 includes grooves 721, 722 in positions respectively corresponding to the recesses 711, 712. Each of the first and second syringes 2, 3 possesses a diameter-reduced portion 22 at its forward end. To mount the first and second syringes 2, 3 in the sprayer, the diameter-reduced portion 22 of the first syringe 2 is received in the groove 721, while the diameter-reduced portion 22 of the second syringe 3 is received in the groove 722.

A rear plate 73 is provided at the rear of the base 71. The rear plate 73 is formed with recesses 731, 732 at positions respectively corresponding to the recesses 711, 712 of the base 71. To mount the first and second syringes 2, 3, the flange 23 (base) of the first syringe 2 engages or is inserted into the recess 731 while the flange 23 (base) of the second syringe 3 engages or is received in the recess 732.

In the sprayer body 7, first and second syringes 2, 3 can be positively fixed side by side in this manner by engaging the diameter-reduced portions 22 with the front plate 72 and by engaging the flanges 23 with the rear plate 73.

Two handles 751, 752 are provided in the vicinity of the rear plate of the base 71. The handles 751, 752 are configured in a manner allowing the operator to put his/her fingers thereon during use of the sprayer 1. One of the handles 751 is constructed as an upwardly protruding or extending plate piece, while the other handle 752 is constructed as a downwardly extending or protruding plate piece. The front surface of each handle 751, 752 which faces to the front is arcuate in shaped, possessing a curved convex form.

Various parts constituting the sprayer body 7 may be formed integrally or may be constructed separately one from another.

The material forming the sprayer body 7 is not particularly limited, but can, for example, be a metal material or plastic. Such materials can be used individually or in combination.

A manipulator 8 is arranged in the rear of the sprayer body 7 and is movable in the longitudinal direction with respect to the sprayer body 7. The manipulator 8 is a part that pushes the plungers 26, 26 of the first and second syringes 2, 3 in the forward direction toward the front (i.e., in the direction of the arrow C in Figs. 1, 2 and 4). The manipulator 8 has a coupler 81 that couples together the flanges 29 of the plungers 26 of the first and second syringes 2, 3, the presser 82 located rearwardly of the coupler 81, and the rail 83 extending from the coupler 81 toward the front.

The coupler 81 is provided with recesses 811, 812 both of which open upwardly. The recess 811 is in a form corresponding to the flange 29 of the plunger 26 of the first syringe 2 so that such flange 29 can be engaged therewith as shown in Fig. 2. Meanwhile, the other recess 812 is in a form corresponding to the flange 29 of the plunger 26 of the second syringe 3 so that the flange 29 of the plunger 26 of the second syringe 3 can be engaged therewith as seen in Fig. 2.

By way of the coupler 81, positive coupling and fixing exists between the flanges 29 of the plungers 26 of the first and second syringes 2, 3. Accordingly, the plungers 26 can be moved together at one time in the direction indicated by the arrow C.

The coupler 81 is provided with a cylindrical portion 813 forming a cylinder located between the one recess 811 and the other recess 812. The cylindrical portion 813 is positioned such that the axis of the cylindrical portion 813 is vertically oriented or is parallel with the vertical direction as shown in Figs. 1 and 2. The cylindrical portion 813 receives the valve means 9.

An elongate rail 83 extends from the outer periphery of the cylindrical portion 813 of the coupler 81 in the forward direction (i.e., towards the front). The rail 83 is provided in the base 71 of the sprayer body 7 and is inserted or positioned in the guide 713 formed in an elongate form. By pushing the manipulator 8 in the direction of the arrow C, the rail 83 is guided along the guide 713. This allows for a relatively smooth pushing operation.

A plate-like pusher 82 is provided a the rear of the cylindrical portion 813 of the coupler 81. The plate-like pusher 82 is adapted to move lengthwise of the sprayer body 7.

The pusher 82 is a part to be pressed by the operator upon using the sprayer 1, i.e. upon applying a mixed liquid to a diseased region, etc. When using the sprayer 1, the user's index finger is put on the handle 751, the middle finger is placed on the handle 752 and the thumb is put on the pusher 82. This makes it possible to positively grip the sprayer 1 in a relatively stable manner. At the same time, the manipulator 8 (pusher 82) can be operated with smoothness and positiveness, thus improving the operability of the sprayer 1.

The pusher 82 is coupled to a second connecting portion 92 of the valve means 9, discussed in more detail below. The material forming the manipulator 8 is not limited to a particular material. Examples include materials such as those listed in the explanation of materials which can be employed in the application body 7.

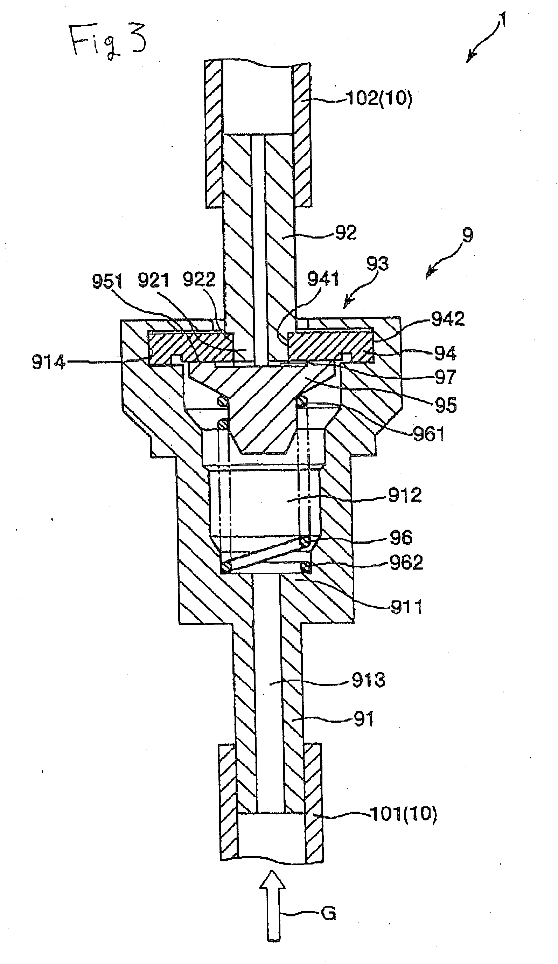

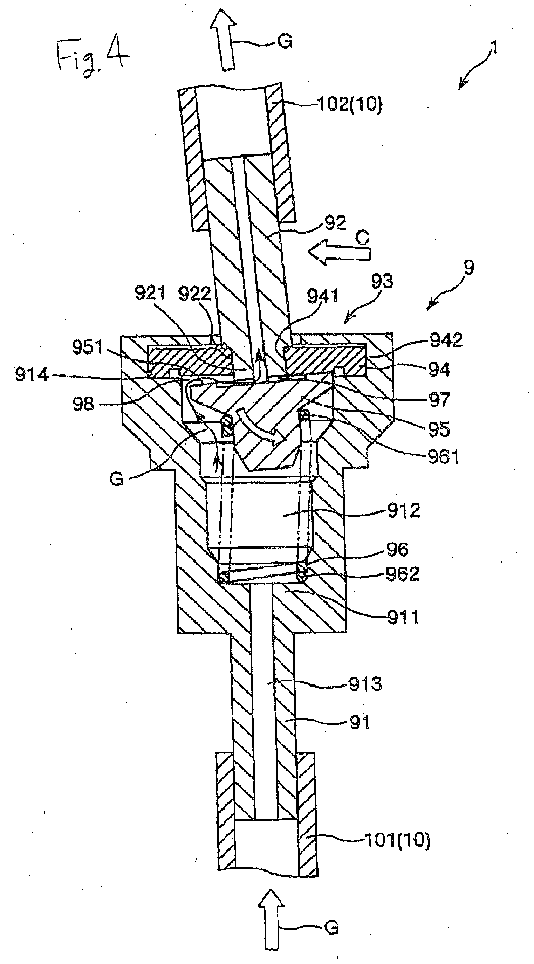

As mentioned before, the valve means 9 is provided in cylindrical portion 813 of manipulator 8. The valve means 9 operates to open and close the gas G flow from the bombe 300 to the nozzle 4. Through the valve means 9, the first tube 101 and the second tube 102 are shut off as seen in Fig. 3 and communicated together as seen in Fig. 4 by the operation of the valve means 9.

As shown in Figs. 3 and 4, the valve means 9 has a first connecting portion 91 connected to the first tube 101, a second connecting portion 92 connected to the second tube 102, and an openable and closable valve 93 received in the first connecting portion 91.

The first connecting portion 91 is tubular in form and has a lumen. The lumen of the first connecting portion 91 includes a container portion 912 (larger-diameter portion), located on the downstream side, and the valve 93. The lumen of the first connecting portion 91 also includes a diameter-reduced portion 913 having a diameter smaller than the inner diameter of the container portion 912 on the upstream side. A step 911, whose inner diameter sharply changes, is formed at the boundary between the diameter-reduced portion 913 and the container portion 912.

The second connecting portion 92 is tubular in form. As mentioned before, the second connecting portion 92 is coupled to the pusher 82 of the manipulator 8. The second connecting portion 92, whose lower end 921 is supported by the seal member 94 of the valve 93 (i.e., the lower end of the second connecting portion is received in a hole in the seal member 94), is arranged downstream of the first connecting portion 91 through the seal member 94. The second connecting portion 92 is arranged to be displaced between a first position (i.e., the state shown in Fig. 3) in which the axis of the second connecting portion 92 is in alignment relative to the axis of the first connecting portion 91 (i.e., the first and second connecting portions are coaxial) and a second position (i.e., the state shown in Fig. 4) in which the axis of the second connecting portion 92 is inclined in the direction of the arrow C (the operating direction) of the pusher 82 (manipulator 8) about the lower end 921. In this second position of the second connecting portion 92, the axis of the second connecting portion 92 is not in alignment relative to the axis of the first connecting portion 91 (i.e., the first and second connecting portions are not coaxial).

The seal member 94 of the valve 93 is constructed as an elastic material. The valve 93 includes the seal member 94, a flange 95 positioned upstream of the seal member 94, and a bias member 96 that biases the flange 95 toward the seal member 94.

The seal member 94 is in a link form, and has an inner periphery 941 in close contact with the outer periphery 922 of the lower end 921 of the second connecting portion 92. The seal member 94 has an outer periphery 942 in close contact with the inner periphery 914 of the container 912 of the first connecting portion 91. With the seal member 94 thus constructed, the first connecting portion 91 and the second connecting portion 92 are hermetically coupled together through the seal member 94.

The flange 95 has an outer diameter greater than the outer diameter of the second connecting portion 92. The flange 95 is arranged oppositely to (in facing relation to) a lower end surface of the second connecting portion 92 through gap 97.

In this illustrated embodiment, the bias member 96 is a compression coiled spring. In the compressed state, its upper end 961 is in abutment against the flange 95 while the lower end 962 is in abutment against the step 911. This can positively bias the flange 95 toward the seal member 94.

With this illustrated and described embodiment of the valve 93, the flange 95 is biased by the bias member 96 and placed in hermetic contact with the seal member 94 as shown in Fig. 3 when the second connecting portion 92 is in a first position, i.e. when no external force is applied to the second connecting portion 92. This places the valve 93 in the closed state.

When a press force is applied in the direction of the arrow C to the second connecting portion 92 by the presser 82 of the manipulator 8, the second connecting portion 92 is displaced or moved from the first position into the second position. At this time, the flange 95 is displaced against the bias force of the bias member 96. Due to this, at least a part of the periphery 951 of the flange 95 moves away from the seal member 94 to create a gap 98 with the seal member as seen in Fig. 4. Due to this, gas G flows from the first connecting portion 91 to the second connecting portion 92 through the gap 98. In other words, the valve 93 is positioned in the open state.

The valve means 9 constructed by way of example in this way allows the valve 93 to positively open and close interactively with the pressing of the manipulator 8. By virtue of this, when the valve 93 is in the closed state, the gas G is positively shut off from flowing from the bombe 300 to nozzle 4. When the valve 93 is in the open state, the gas G can be positively released to flow.

The material forming the first connecting portion 91, the second connecting portion 92, the flange 95 and the bias member 96 is not limited, but can include, for example, metal material or plastic, either individually or in combination.

The material of which the seal member 94 is made is also not limited to a specific material. Examples of suitable materials include rubber material of various types, e.g. natural or butyl rubber.

The nozzle 4 is arranged on the front plate 72 of the sprayer body 72. The nozzle 4 ejects the gas G passing through the tube 10, the first liquid L1 passing through the diameter-reduced portion 22 of the first syringe 2, and the second liquid L2 passing through the diameter-reduced portion 22 of the second syringe 3. As shown in Fig. 1, the nozzle 4 includes a nozzle body 41, a nozzle head 42 positioned forward of or in front of the nozzle body 41, and a connecting portion 43 connected between the nozzle body 41 and the nozzle head 42.

By way of example, the nozzle body 41 is constructed of a metal material or a resin material. The nozzle body 41 has a hollow region opening at its front and rear ends. With this construction of the nozzle body 41, the base opening 411 is adapted to engage the front plate 72 of the sprayer body 7. This fixes the nozzle 4 to the sprayer body 7.

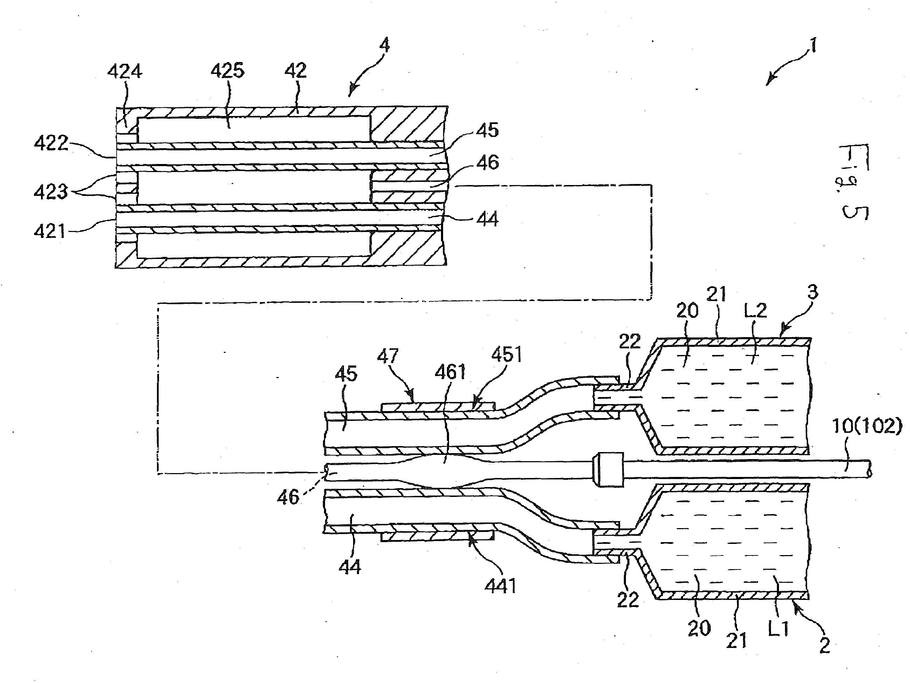

As shown in Figs. 5-9, the nozzle head 42 is circularly cylindrical in outer form. The inside of the nozzle head 42 is provided with a hollow region 425. The hollow region 425 has a pair of adjacent openings that open in the tip wall 424. Each of the openings serve as a third orifice (gas orifice) 423 through which the gas G in the hollow region 425 is ejected.

The material of the nozzle head 42 is not limited to a particular material. It is possible to use the same materials as the structural materials of the nozzle body 41.

As shown in Fig. 1, the connecting portion 43 is constructed as an elongate tubular body, connecting the tip opening 412 of the nozzle body 41 and the base end of the nozzle head 42. The connecting portion 43 may be constructed of a rigid material or a non-rigid material, an elastic material or the like, which may have flexibility. The material forming the connecting portion 42 may be polyvinyl chloride, polyethylene, polypropylene, stainless steel, aluminum or the like.

The connecting portion 43 serves also as a protection member that protects three tubes, described in more detail below, receiving through the same.

Three tubes pass through the nozzle body 41 and the connecting portion 43. The three tubes include one defining a first passage 44 for feeding the first liquid L1 toward the nozzle head 42, another one defining a second passage 45 for feeding the second liquid L2 toward the nozzle head 42, and a third one defining a third passage 46 for feeding the gas G from the bombe 300 toward the nozzle head 42. Those tubes are each constructed of a non-rigid resin material. The non-rigid resin material need not be any specific material. Examples of suitable materials include polyethylene, polyvinyl chloride, polybutadiene, polyamide, polyester, silicone, polyurethane or the like.

The first, second and third passages 44, 45, 46, defined by such tubes, have base ends connected in a liquid-tight manner to the diameter-reduced portion 22 of the first syringe 2, the diameter-reduced portion 22 of the second syringe 3 and the second tube 102 of the tube 10, respectively.

The tube forming the first passage 44 is inserted into the nozzle head 42 so that the passage communicates with a tip opening in the tip wall 424. The opening in the tip wall 424 serves as first orifice (liquid orifice) 421 for ejecting the first liquid L1. The first orifice 421 is arranged concentric to a third orifice 423 that is one of two third orifices 423 (the lower one in Fig. 5). The first liquid L1 is ejected at the first orifice 421 through the diameter-reduced portion 22 of the first syringe 2 and the first passage 44 in that order.

The tube forming the second passage 45 is inserted into the nozzle head 42 in a manner similar to the first passage 44 so that the second passage communicates with a tip opening in the tip wall 424. The opening in the tip wall 424 serves as a second orifice (liquid orifice) 422 at which the second liquid L2 is ejected. The second orifice 422 is arranged concentric to a third orifice 423 that is the other (the upper one in Fig. 5) of the two third orifices 423. The second liquid L2 is ejected at the second orifice 422 through the reduced-diameter portion 22 of the second syringe 3 and the second passage 45 in that order.

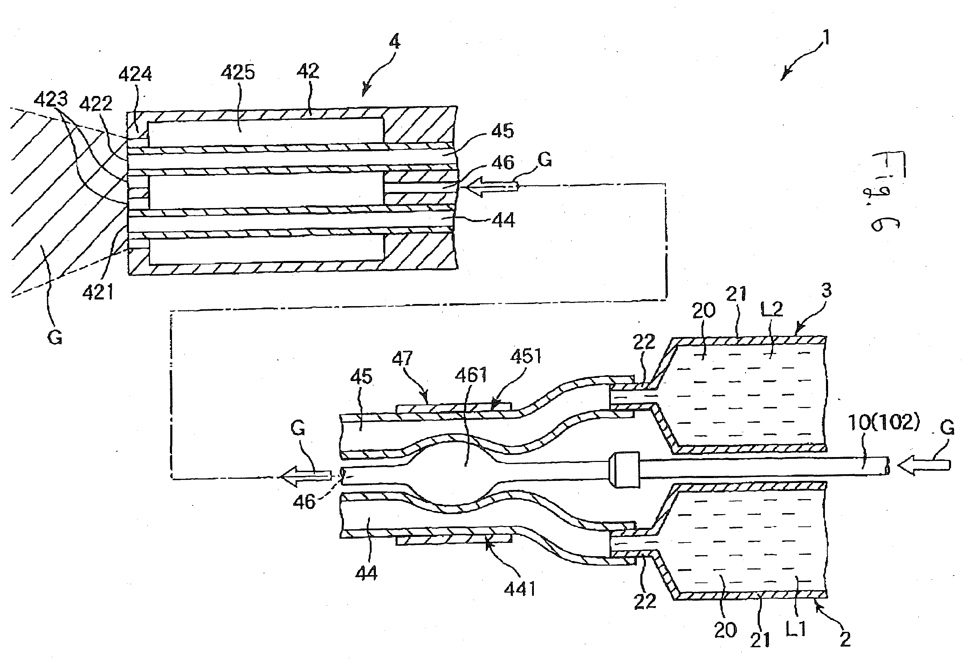

As shown in Fig. 5 (similarly in Figs. 6-9), the first orifice 421 and the second orifice 422 are equal in size to each other. Meanwhile, the two third orifices 423 are equal in size to each other, and are arranged around the outer periphery of the first and second orifices 421, 422.

The tip of the third passage 46 is received in the base end of the nozzle head 42 and opens to the hollow region 425. In the open state of the valve 93 (valve means 9), gas G flows from the bombe 300 and through the tube 10, the third passage 46 and the hollow region 425 in that order, and is ejected at the third orifices 423.

The tube 10 is comprised of a first tube 101 located upstream (closer to the bombe 300) with respect to valve means 9 and a second tube 102 located downstream (closer to the nozzle 4). The material forming the tube 10 (first tube 101 and second tube 102) is not especially limited. By way of example, materials similar to those described above for the tube defining the first, second and third passages 44; 45, 46.

When the manipulator 8 is operated, gas G ejects at high speed through the third orifice 423. The first liquid L1 ejected at the first orifice 421 and the second liquid L2 ejected at the second orifice 422 are entrained (mixed) in the gas G that is ejected at high speed. At this time, the first liquid L1 and the second liquid L2 are ejected in a spray form. Due to this, the first liquid L1 and the second liquid L2 are positively mixed together and applied to the diseased region.

As shown in Figs. 5-9, a volume changer or volume changing portion 441 is provided on the first passage 44 in a position close to the first syringe 2 to change the interior volume or capacity of the first passage. The volume changer 441 can serve as a passage forming region constituting a part of the relevant first passage 44. For example, where the volume changer 441 is formed integral with the first passage 44, the nozzle 4 is relatively easy to fabricate.

A volume changer or volume changing portion 451 is provided on the second passage 45 in a position close to the second syringe 3 to change the interior volume thereof in a manner similar to the first passage 44. The volume changer 451 can serve as a passage forming region constituting a part of the relevant second passage 45. For example, where the volume changer 451 is formed integral with the second passage 45, the nozzle 4 is relatively easy to fabricate.

The volume changers 441, 442 are symmetrically arranged in position about the third passage 46.

An inflatable portion 461 is provided on the third passage 46 and is adapted to expand and contract depending upon the flow rate of the gas G. Namely, the inflatable portion 461 is arranged between the volume changer 441 and the volume changer 451. The inflatable portion 461, arranged in such a position, can be provided as a portion of the tube wall that is thinner than the adjoining portions. This allows the inflatable portion 461 to expand when the gas G is supplied through the tube and to contract into the former form when the gas G ceases from being supplied. The inflatable portion 461 serves as a deform means that presses and deforms the volume changers 441, 451 in a manner increasing and decreasing the capacities of the volume changers 441, 451. In the illustrated embodiment, the inflatable portion is a balloon. The inflatable portion 461 also operates as a pressure applicator or pressure applying means that applies pressure (variable pressure) to the volume changers 441, 451 to change the volume of the volume changers.

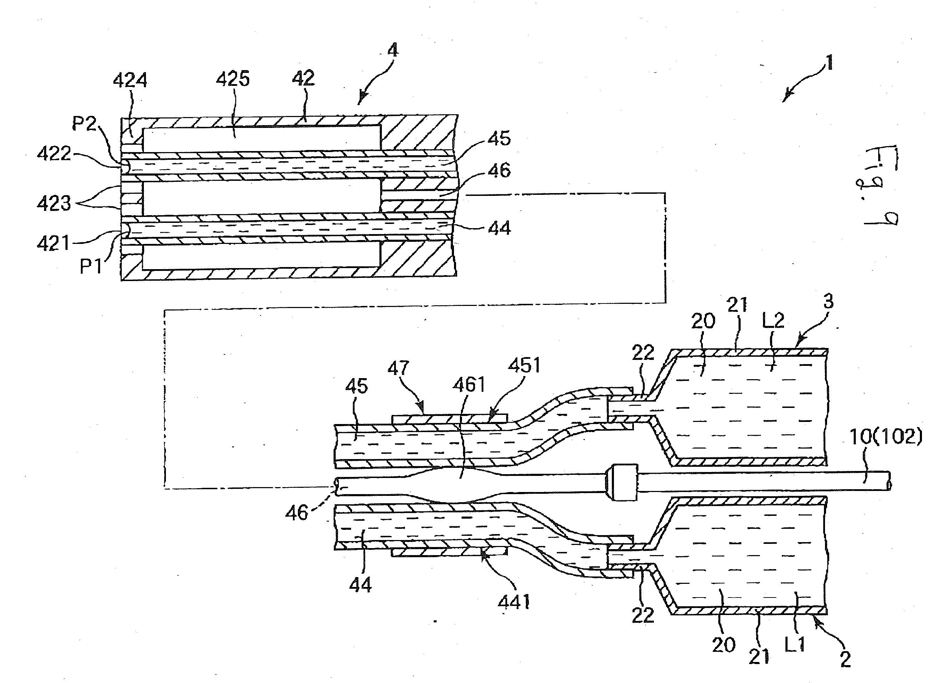

As shown in Figs. 5 and 9, the inflatable portion 461 in a natural state has an outer diameter somewhat greater than the outer diameter of the passage 46 in adjoining positions. In this state, the inflatable portion 461 may be in contact with the volume changers 441, 451 to a degree not pressing those or be departed from the volume changers 441, 451. In the structure shown in Figs 5 and 9, inflatable portion 461 is in contact with the volume changers 441, 451 to a degree not pressing them. In this case, the volume changers 441, 451 are at the maximum capacity.

When gas is supplied through the third passage 46, the inflatable portion 461 is expanded by being supplied with gas as shown in Figs. 6-8. The inflatable portion 461 thus expanded presses the volume changers 441, 451 against the elastic force of the volume changers 441, 451. This reduces the respective capacities (volume or interior dimension) of the volume changers 441, 451. The inflatable portion 461 thus expanded presses the volume changer 441 to a degree which does not place the inner surface of the volume changer 441 into close contact. That is, the passage 44 is not completely closed. The same is also true for the volume changer 451.

When the expended inflatable portion 461 contracts, the volume changers 441, 451 are released from being pressed. Due to this, the volume changers 441, 451 are restored in shape by their own elasticity as shown in Fig. 9.

Meanwhile, the nozzle 4 has a ring (band) 47 holding the volume changers 441, 451 and the inflatable portion 461 collectively from their outer confines. The ring 47 is formed by winding a strip made of, for example, plastic material. The ring 47 restricts the positional relationship between the volume changers 441, 451 and the inflatable portion 461 irrespective of the expansion and contraction of the inflatable portion 461. Due to this, when the inflatable portion 461 expands, the expanded inflatable portion positively presses the volume changers 441, 451.

In this manner, in the sprayer 1, the capacities of the volume changers 441, 451 positively change depending upon the expansion and contraction of the inflatable portion 461.

The following is a description of the sprayer 1 in a usable state, i.e. in a state where the first and second syringes 2, 3 are respectively filled with the first and second liquids L1, L2 and connected to the gas bombe 300.

The first and second syringes 2, 3 are respectively filled with the first and second liquids L1, L2 in amounts needed to be applied to the diseased region. Meanwhile, the bombe 300 is opened at its valve 301 and allowed to supply gas G to the sprayer 1.

In the sprayer 1, the force causing a gap 98 between the seal member 94 and the flange 95 which operates against the force of the bias member 96 urging the flange 95 on the seal member 94, i.e. the pressing force to incline the second connecting portion 92 in the direction of the arrow C from the first position into the second position, is established smaller than the force that moves the plungers 26 of the first and second syringes 2, 3 toward the front. Namely, before the movement of the plungers 26 occurs, the gap 98 arises to supply gas G. Such setting is available by properly establishing various conditions, for example, the spring constant of the bias member 96, the liquid viscosities of the liquids and the inner diameters of the outer shells 21.

In use, the user's index finger is put on the handle 751 of the sprayer body 7, the user's middle finger is put on the handle 752 and the user's thumb is placed on the presser 82 of the manipulator 8. At this time, the first liquid L1 is not supplied to the first passage 44, the second liquid L2 is not supplied to the second passage 45, and the gas G is not supplied to the third passage 46 as shown in Fig. 5. Due to this, the gas G and the first and second liquids L1, L2 are not ejected through the nozzle 4. In addition, because the inflatable portion 461 is not expanded, the volume changers 441, 451 are not pressed by the inflatable portion.

In this state, when the presser 82 is pushed by the thumb, the second connecting portion 92 first inclines to cause the gap 98 between the seal member 94 and the flange 95. The gas G passes through the gap 98 as generally shown in Fig. 4. This supplies gas G to the third passage 46 through the second tube 102, thus ejecting gas G through the third orifices 423 of nozzle 4 as seen in Fig. 6. At this time, because the inflatable portion 461 expands, the volume changers 441, 451 are pressed. The expansion of the inflatable portion 461 is maintained until terminating the supply of gas to the inflatable portion 461.

Meanwhile, the pushing of the presser 82 with the thumb does not yet move the manipulator 8 in its entirety, i.e. the plungers 26 are not yet moved toward front. Accordingly, first and second liquids L1, L2 are not yet supplied respectively to the first and second passages 44, 45.

When the presser 82 is pushed further, the second connecting portion 92 is inclined to such an extent that the press force of the thumb is delivered to the coupler 81 through the presser 82. This causes the coupler 81 (the manipulator 8 in its entirety) to begin moving, thereby pushing the first liquid L1 out of the first syringe 2 and the second liquid L2 out of the second syringe 3. The pushed out portion of the first liquid L1 passes through the volume changer 441 that remains pressed by the inflatable portion 461 and moves toward the front, i.e. it reaches the first orifice 421 where it is ejected therefrom as indicated in Fig. 7. Meanwhile, the second liquid L2 passes through the volume changer 451 which remains pressed by the inflatable portion 461 and reaches the second orifice 422 where it is ejected therefrom as shown in Fig. 7, in a manner quite similar to the first liquid L1.

The first and second liquids L1, L2 thus ejected take spray forms and are mixed with each other, thus being applied to a diseased region of the body.

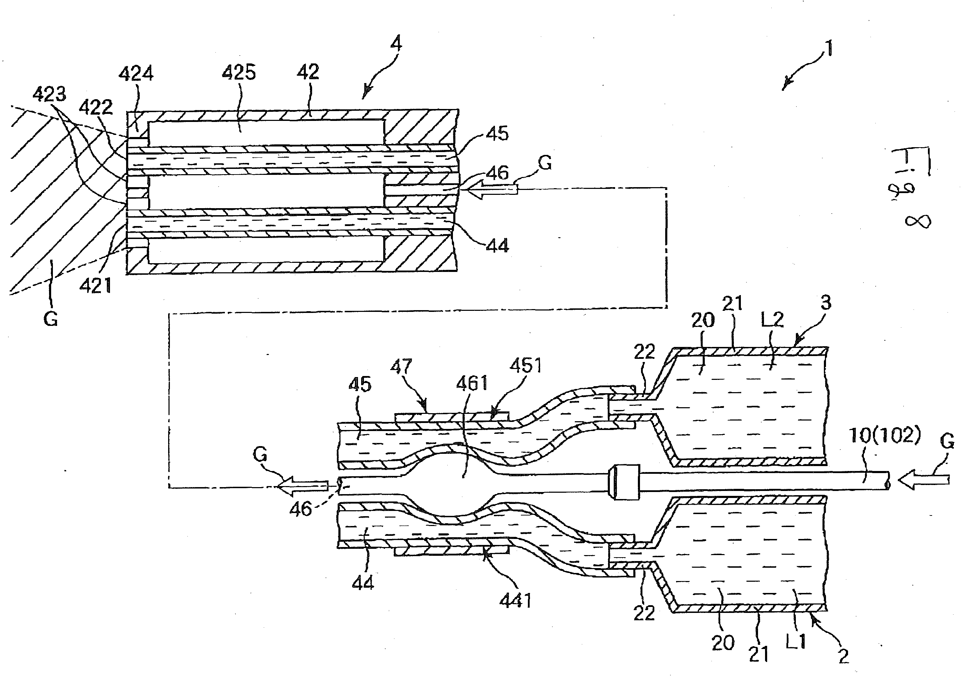

After completing a predetermined amount of application to the diseased region, the entire manipulator 8 stops moving as the pressing force of the thumb on the presser 82 (manipulator 8) is reduced. As generally shown in Fig. 8, this stops the plungers 26 from moving and hence stops the first and second liquids L1, L2 from ejecting. At this time, because the second connecting portion 92 is kept in a second position by the urging of the presser 82, the gas G continues to be ejected as shown in Fig. 8.

As the urging force of the thumb on the presser 82 is reduced, the thumb urging presser 82 ultimately moves away from the presser 82. Due to this, the urging force applied to the second connecting portion 92 is canceled or ceases and so the second connecting portion 92 returns to the first position. This eliminates the gap 98 between the seal member 94 and the flange 95. That is, the seal member 94 and the flange 95 resume positions at which the entire outer periphery 951 of the flange is placed in close contact with the seal member 94. At this time, the gas G ceases from being supplied to the third passage 46 and hence gas G also ceases from being ejected at the third orifices 423 as shown in Fig. 9.

When the gas G ceases from being supplied to the third passage 46, the inflatable portion 461 contracts as indicated in Fig. 9. This cancels the pressing of the inflatable portion 461 on the volume change portions 441, 451, thus increasing the capacities of the volume changers 441, 451 greater than those in the state where the first and second liquids L1, L2 are being ejected. Thus, as shown in Fig. 9, by increasing the volumes, the first liquid L1 is rearwardly retracted toward the rear at its tip P1 rather than being urged towards the first orifice 421. Similarly, the second liquid L2 is rearwardly retracted toward the rear at its tip P2 rather than being urged toward the second orifice 422.

By thus retracting the first and second liquids L1, L2 at the respective front ends P1, P2, the first and second liquids L1, L2 are prevented from being mixed together into gelatination in the vicinity of the first or second orifice 421, 422. This makes it possible to positively inhibit or prevent clogging otherwise caused in the vicinity of the first and second orifices 421, 422 after use of the sprayer 1, i.e. after application. The sprayer 1 in a state free of clogging can be used again in the application to a diseased region.

Meanwhile, the sprayer 1 can optimally set up the timing of supplying and ceasing from supplying the gas G to the nozzle 4 in the case of ejecting, or stopping from ejecting, the first and second liquids L1, L2 through the nozzle 4. In other words, the sprayer 1 is structured to eject the gas G through the nozzle 4 with relative ease and positiveness in advance of the first and second liquids L1, L2.

This can help prevent only the first and second liquids L1, L2 from being ejected and applied to a diseased region. In addition, owing to the ejected portion of the gas G, the first and second liquids L1, L2 are positively ejected in respective spray forms and hence those liquids are positively mixed with each other.

The sprayer 1 is constructed to stop the gas G from being ejected at a timing later than the first and second liquids L1, L2. Due to this, where liquids remain (put on) at the orifices because of the stopping of the liquids from ejecting, the remaining liquid can be blown away by the gas G. Therefore, it is possible to positively prevent the clogging at the orifices due to the disadvantage, i.e. gelatination as caused by the reaction of the first and second liquids at the orifices.

In this embodiment, the inflatable portion 461, operating under the supply of the gas G from the source 300, form a deformer that deforms the volume changer or volume changing portions 441, 451 in the manner described above.

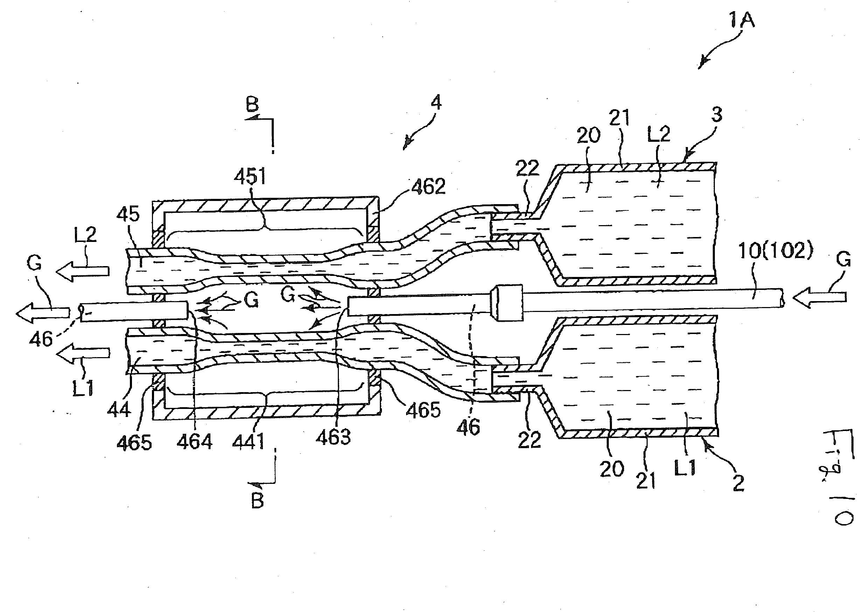

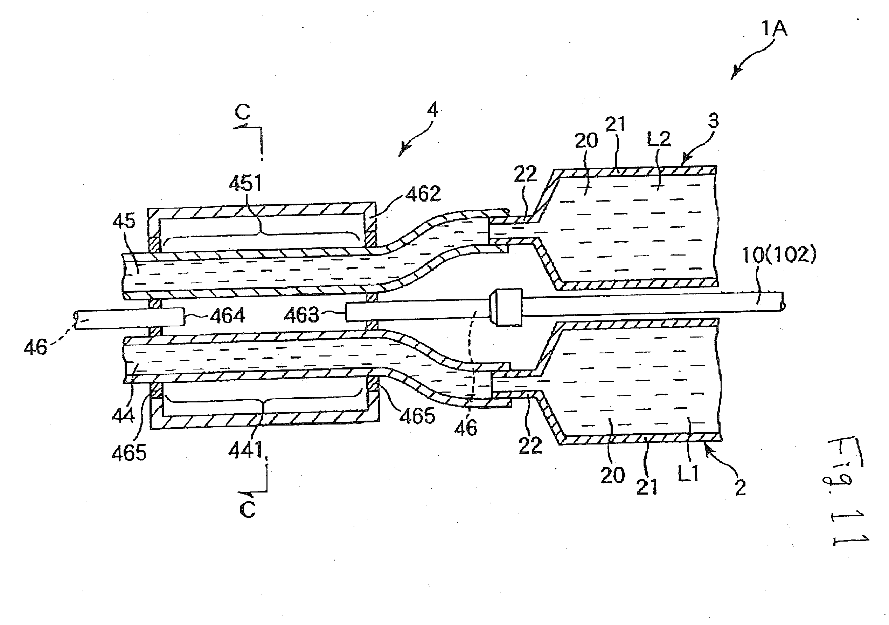

Figs. 10 and 11 illustrate portions of a second embodiment of a sprayer illustrating chronologically a change in application state. The following describes the second embodiment of the sprayer disclosed herein. The discussion below primarily considers aspects of the sprayer that differ from the embodiment described above. Features in this second embodiment that are the same as those associated with the first embodiment are designated by the same reference numerals and a detailed discussion of such features is not repeated.

This embodiment is similar to the first embodiment except that this embodiment employs a different deform means.

In the sprayer 1A shown in Figs. 10 and 11, the pressurizing chamber 462 is provided on the third passage 46. The tube defining the third passage 46 is cut within the pressurizing chamber 462 so that the open ends 463, 464 open in the pressurizing chamber 462. This allows gas G to enter the pressurizing chamber 462 at one opening 463 (on the right side in Fig. 10). The gas G is temporarily stored in the pressurizing chamber 462 and then exits at the other opening 464 (on the left side in Fig. 10). By storing the gas G in the pressurizing chamber 462, the interior pressure of the pressurizing chamber 462 increases.

The volume changers or volume changing portions 441, 451 are located within the pressurizing chamber 462. The pressurizing chamber 462 is in a cylindrical form, having both ends connected in a liquid-tight manner to the volume changers 441, 451 through rubber packings 465.

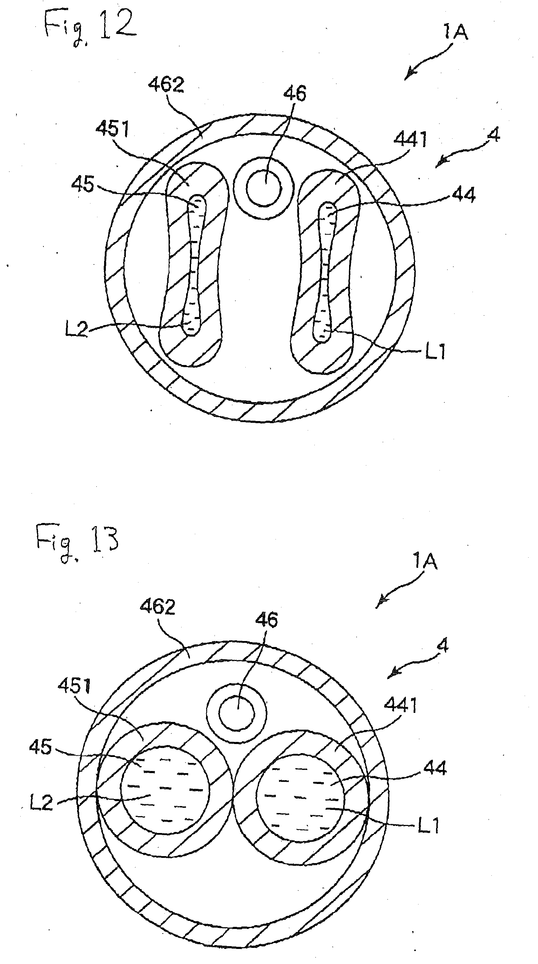

In the sprayer 1A thus constructed, by increasing the interior pressure of the pressurizing chamber 462 as noted before, the volume changers 441, 451 are pressed as generally shown in Figs. 10 and 12. On the other hand, ceasing (decreasing) the supply of gas G to the pressurizing chamber 462 and changing the increase of the interior pressure of the pressurizing chamber 462 into a decrease in pressure, the pressing force or pressure applied to the volume changers 441, 451 is reduced or cancelled as generally shown in Figs. 11 and 13.

Accordingly, the pressurizing chamber 462 serves as a deformer or deform means for changing the form or volume of the volume changers 441, 451 in conjunction with the gas G supplied from the source. The pressurizing chamber also serves as a pressure applicator or pressure applying means that applies pressure (variable pressure) to the volume changers 441, 451 to change the volume of the volume changers.

The interior pressure of the pressurizing chamber 462 is capable of being increased, though is preferably limited so that the pressure does not increase above a level causing the inner peripheral surface regions of the volume changers 441, 451 to contact each other. That is, the pressure applied by the pressurizing chamber is preferably limited so as not to close off the passages in the volume changers.

As shown in Figs. 10 and 11, as well as Figs. 12 and 13, the capacities of the volume changers 441, 451 when the first and second liquids L1, L2 cease from being ejected (i.e., in the state shown in Figs. 11 or 13) are greater than that when the first and second liquids L1, L2 are being ejected (i.e., in the state shown in Figs. 10 or 12). As a result, the first liquid L1 at its front end is retracted toward the rear rather than possibly being ejected in the forward direction through the first orifice 421, while the second liquid L2 at its front end is retracted toward the rear rather than possibly being ejected in the forward direction through the second orifice 422. Accordingly, the first and second liquids L1, L2 are prevented from being mixed into gelatinization in the vicinity of the first or second orifice 421, 422. As a result, clogging can be positively inhibited or prevented from occurring in the vicinity of the first and second orifices 421, 422 after the use of the sprayer 1.

In the arrangement shown in Figs. 10 and 11, the volume changers 441, 451 are arranged in the same position or orientation as the lengthwise direction of the nozzle 4. However, this arrangement is not required as the volume changers may be arranged, for example, in different positions with respect to the lengthwise extent of the nozzle 4.

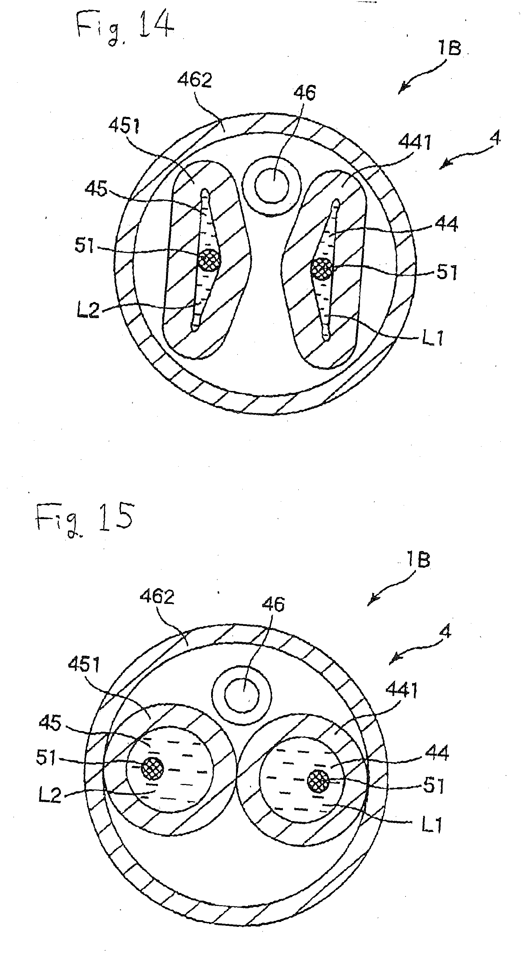

Figs. 14 and 15 illustrate a nozzle of a sprayer according to a third embodiment, illustrating a chronological change in application state. Fig. 14 illustrates a state in which the liquid is being ejected while Fig. 15 illustrates a state in which the liquid is no longer being ejected. The following description of the third embodiment primarily considers aspects of the sprayer that differ from the embodiments described above. Features in this third embodiment that are the same as those associated with the embodiments already described are designated by the same reference numerals and a detailed discussion of such features is not repeated.

This third embodiment is similar to the second embodiment, except that the sprayer is provided with passage-securing means.

The sprayer 1B shown in Figs. 14 and 15 includes two spacers 51 serving as passage-securing means. Each of the spacers 51 is an elongate member which can be in the form of a wire. The elongate member can be constructed, for example, of a metal material and passing through the respective volume changer or volume changing portion 441, 451. The spacers 51 are fixed in position in the volume changers 441, 451 respectively by appropriate fixing members. Each of spacers 51 is circular in cross-section in the illustrated embodiment. However, the spacers are not limited in this regard as they may take on other shapes such as elliptic, oval or polygonal. The spacers 51 preferably extends the entire longitudinal extent of the volume changers 441, 451.

Because the two spacers 51 can have the same construction and function similarly, the spacer 51 received through the volume changer 441 is explained representatively, it being understood that the same description applies to the spacer in the other volume changer 451.

When the volume changer 441 is pressed to decrease its capacity or volume by increasing the interior pressure in the pressurizing chamber 462, the spacer 51 helps prevent portions of the inner surface of the volume changers 441 from contacting each other as generally shown by way of example in Fig. 14. This helps maintain the volume changer in an open state and helps secure (maintain) the passage of the first liquid L1 through the volume changer 441. Accordingly, the first liquid L1 is able to be positively ejected at the first orifice 421. Stated differently, the spacer 51 advantageously helps prevent the volume changer from being completely closed off in a manner that does not permit the passage of the first liquid L1.

Even where the interior pressure of the pressurizing chamber 462 increases to a degree that parts of the inner peripheral surface of the volume changer 441 contact one another, the spacer 51 helps ensure that the volume changer is not completely closed and that a gap still positively exists by virtue of the spacers 51. In this manner, even where the volume changer 441 is pressed excessively (decreased in volume excessively), the first liquid L1 is open to permit the passage of the first liquid L1 through the volume changer 441.

Also, because the spacer 512 helps prevent the inner surface of the volume changer 441 from contacting itself even in the ejection state, the inner peripheral surface regions of the volume changer 441 swiftly separate after completion of the ejection. That is, the volume changer 441 is relatively swiftly restored as generally shown in Fig. 15.

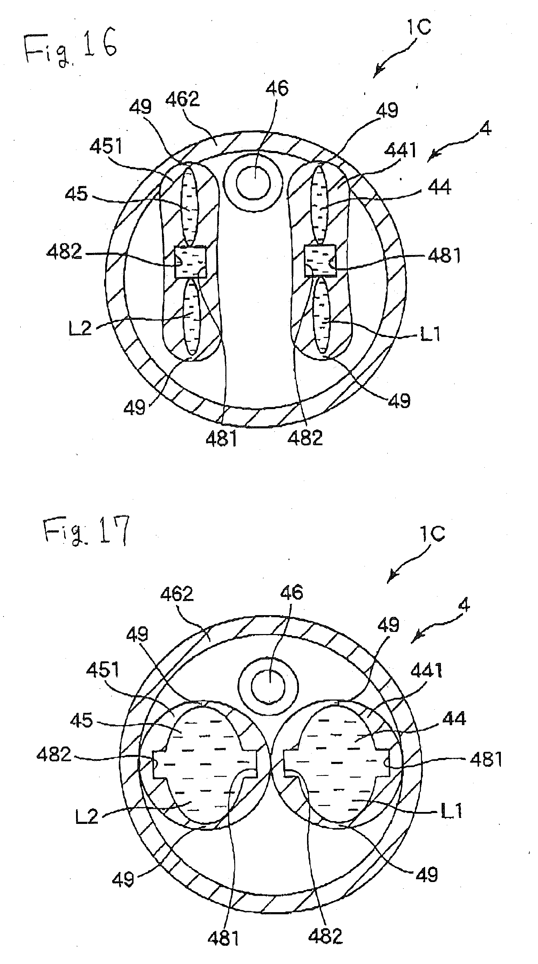

Figs. 16 and 17 show the nozzle of a sprayer according to a fourth embodiment, illustrating chronologically a change in application state in which Fig. 16 illustrates a state in which the liquid is being ejected while Fig. 17 illustrates a state in which the liquid is ceased from being ejected. The discussion below primarily considers aspects of the sprayer that differ from the embodiments described above. Features in this embodiment that are the same as those associated with the embodiments described above are designated by the same reference numerals and a detailed discussion of such features is not repeated.

This fourth embodiment is similar to the third embodiment except that the passage-securing means in this embodiment is different.

In the sprayer 1C shown in Figs. 16 and 17, two grooves 481, 482 are formed in the inner peripheral surface of each volume changer or volume changing portion 441, 451. As both of the volume changers 441 and 451, and the associated grooves 481, 482 in each volume changer are the same, the description below primarily describes the grooves 481, 482 in one of the volume changers 441, it being understood that the same applies to the other volume changer 451.

The grooves 481, 482 are formed lengthwise of the volume changer 441, meaning the grooves 481, 482 extend along the lengthwise extent of the volume changer 441. As shown in Fig. 17, the grooves 481, 482 are positioned in opposing relation to each other in the state that first liquid L1 is ceased from being ejected.

As shown in Fig. 16, in a state in which the volume changer 441 is decreased in volume, the grooves 481, 482 approach one another, and even though the portions of the inner surfaces of the volume changer 441 surrounding the grooves 481, 482 contact one another, the grooves 481, 482 face each other and create a space. This helps ensure the passage of the first liquid L1 through the volume changer 441. That is, the grooves 481, 482 helps ensure that the volume changer 441 is not able to become completely closed off. Therefore, it is possible to reliably provide for the positive ejection of the first liquid L1 at the first orifice 421.

In this manner, each of the grooves 481, 482 functions as a passage-securing means or passage-maintaining means that secures or maintains the passage of the liquid L1 through volume changer 441.

The tube wall of the volume changers 441 is provided with easy-to-deform portions (restricting means) 49 having a smaller wall thickness than adjoining portions of the tube wall. These easy-to-deform portions 49 are positioned at two points equally circumferentially spaced from one another or at diametrically opposite positions on the tube wall. These portions 49 are portions of the tube wall that are more easily deformed than the adjoining portions of the tube wall. The two easy-to-deform portions 49 are arranged between the grooves 481, 482 with respect to the circumferential of volume changer 441.

When the volume changer 441 is pressed, the easy-to deform portions 49 deform more readily than other portions of the tube wall, and deform before the portions of the tube wall other than the easy-to deform portions 49. This restricts or controls the main direction of deformation of the volume changer 441 when the volume changer 441 is pressed, thus allowing the grooves 481, 482 to move towards each other and hence securing the passage as shown in Fig. 16. Thus, the thinned wall portions defining the easy-to deform portions 49 control the deformation of the volume changer 441 to help ensure that during the application of pressure to the volume changer, the tube of the volume changer 441 deforms in a way causing the grooves 481 to approach one another in a manner which helps ensure that a space remains in the volume changer and that the volume changer does not become completely closed off.

The number of grooves provided in the tube of the volume changer 441 is two in this illustrated embodiment. However, the sprayer is not limited in this regard as more than this number of grooves may be provided.

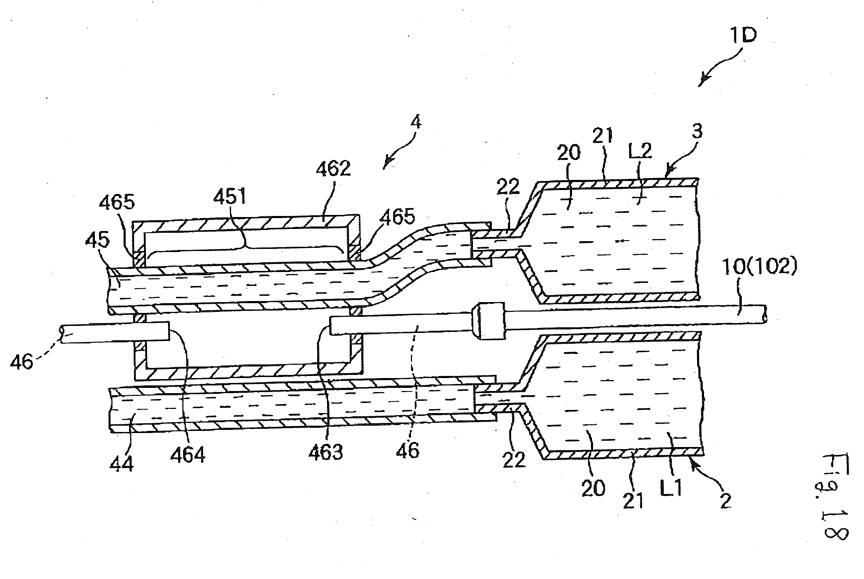

Fig. 18 shows the nozzle of a sprayer according to a fifth embodiment. The description below primarily considers aspects of this fifth embodiment that differ from the embodiments described above. Features in this embodiment that are the same as those associated with the embodiments described above are designated by the same reference numerals and a detailed discussion of such features is not repeated.

This embodiment is similar to the second embodiment described above, except that the arrangement position of the deform means is different.

In the sprayer 1D shown in Fig. 18, only the second passage 45 passes through the pressurizing chamber 462. The volume changer or volume changing portion 451 is constituted by the portion of the second passage 45 passing through the pressurizing chamber 462. The volume changer 451 can be deformed in a manner increasing and decreasing its capacity or volume depending upon the change in the interior pressure of the pressurizing chamber 462. This causes the second liquid L2 at the front end P2 of the passage 45 to move or be drawn toward the rear rather than moving toward the second orifice 422 in a manner similar to the second embodiment.

In the sprayer 1D, even if a situation occurs where the first liquid L1 protrudes at its front end P1 outwardly beyond the first orifice 421 in the non-ejection state (i.e., when the operator intends for the ejection to have stopped), the front end P2 of the second liquid L2 is retracted rearwardly rather than being urged forward to eject from the second orifice 422, thereby preventing the possibility that the first and second liquids L1, L2 will mix together. This can positively inhibit or prevent clogging from occurring in the vicinity of the first and second orifices 421, 422 after the use of the sprayer 1D.

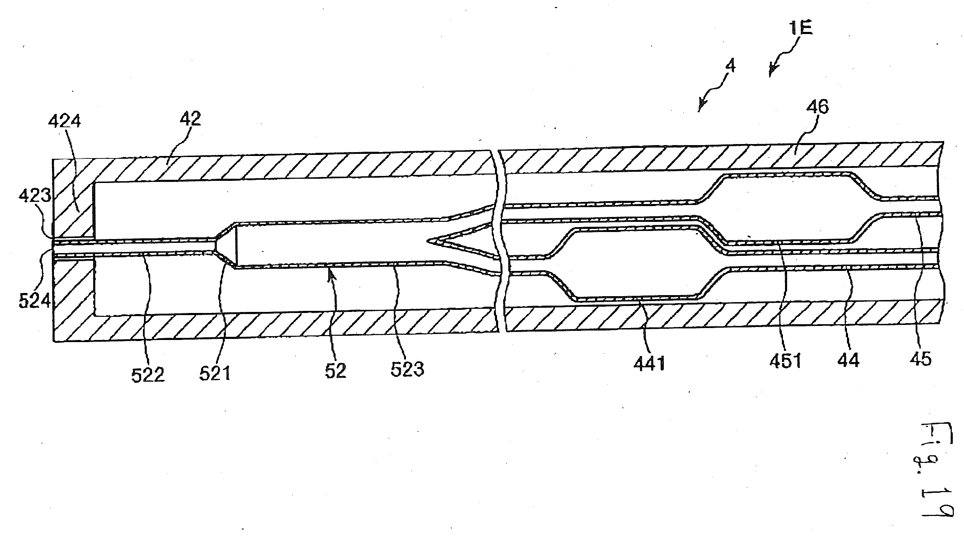

Figs. 19-21 show the nozzle of a sprayer according to a sixth embodiment, illustrating chronologically a change in application state. The description below addresses aspects of the sprayer that differ from the embodiments described above. Features in this embodiment that are the same as those associated with the embodiments described above are designated by the same reference numerals and a detailed discussion of such features is not repeated. For the sake of explanatory convenience, the left and right sides in Figs. 19-21 are referred to as the "front" and "rear (base)" sides.

This sixth embodiment is similar to the second embodiment except that the volume changer possess a different shape and the sprayer further possesses a confluent region.

The sprayer 1E shown in Figs. 19-21 does not include the connecting portion 43 between the nozzle body 41 and the nozzle head 42 as in the earlier embodiment. In this sixth embodiment, the third passage 46 serves as a connecting portion, with the first and second passages 44, 45 passing through the third passage 46.

The nozzle of the sprayer 1E includes a confluence portion 52 where the first liquid L1 passing through the first passage 44 and the second liquid L2 passing through the second passage 45 join together or are mixed. In this confluence portion 52, the first and second passages 44, 45 merge together to form a single passage which continues in the forward direction.

The confluence portion 52 is constructed as a tube communicating with the first passage 44 and with the second passage 45. The material forming the tube is not especially limited, but materials similar to those used to fabricate the tubes forming the first and second passages 44, 45 can be used, for example.

The confluence 52 has a tapering section 521 whose inner diameter decreases in a direction toward the front. By way of this taper, the confluence 52 is divided into a smaller-diameter portion 522 provided closer to the front and smaller in inner diameter and a larger-diameter portion 523 provided closer to the base and larger in inner diameter.

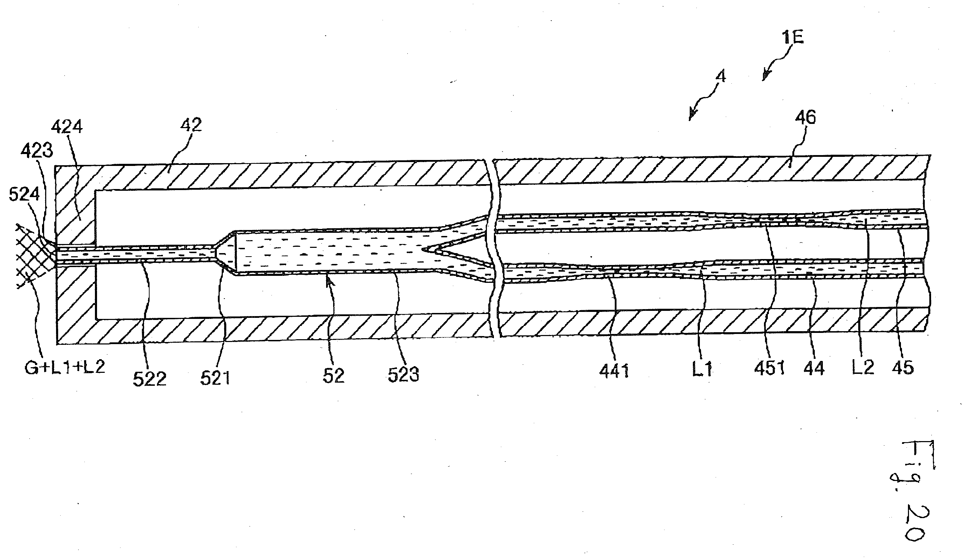

The smaller-diameter portion 522 has a front opening that serves as liquid-mixture orifice 524 through which the liquid mixture of the first and second liquids L1, L2 is ejected as generally shown in Fig. 20. The front end of the smaller-diameter portion 522 is arranged radially inwardly of and concentric to an opening (third orifice 423) provided in the front wall 424 of the nozzle head 42. By virtue of this construction, when gas G is ejected at high speed through the third orifice 423 by operating the manipulator 8, the liquid mixture ejected through the liquid-mixture orifice 524 is entrained in the gas G ejected at high speed. The liquid mixture is thus ejected in a spray form.

With this sprayer 1E, the first liquid L1 and the second liquid L2 join together at the confluence 52 and are mixed together uniformly and positively into a liquid mixture. Then, as mentioned, the liquid mixture is atomized and ejected as a spray of liquid.

One volume changer or volume changing portion 441 is provided on the first passage 44 while the other volume changer or volume changing portion 451 is provided on the second passage 45. Because the volume changers 441, 451 are similar in construction and function, the following description primarily of the volume changer 441 is understood to apply equally to the other volume changer 451.

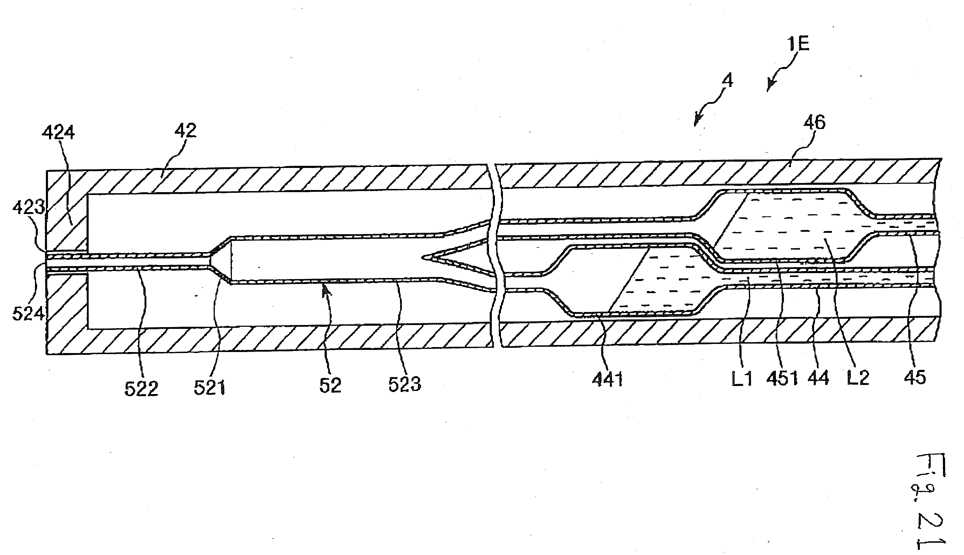

The volume changer 441 is provided as a part of the first passage 44 and in a natural state (i.e., before operation of the sprayer) has an inner diameter greater than the inner diameter of adjoining portions of the first passage 44 (e.g., on either side of the volume changer 441) as shown in Figs. 19 and 21. The volume changer 441 can be in the form of a balloon that is adapted to expand and contract depending upon the pressure within the third passage (i.e., according to changes in pressure applied by the interior gas G of the third passage 46). In the sprayer 1E, the interior pressure in the third passage 46 is increased by introducing the gas G into the third passage 46. Accordingly, in this embodiment of the sprayer 1E, the third passage 46 serves as a pressurizing chamber, preferably over its entire longitudinal extent. The third passage 46 with the gas G supplied from the source operates as a deformer which deforms the volume changing portion(s).

Referring to Figs. 19-21, in a state in which the ejection of the liquid mixture (first and second liquids L1 and L2) ceases (i.e., the state shown in Fig. 19), the volume changer 441, 451 has a capacity greater than that in the state in which the liquid mixture is being ejected (i.e., the state shown in Fig. 20). Due to this, liquid mixture in the confluence 52 is retracted or drawn rearwardly (i.e., into the volume changers 441, 451) rather than tending to move forwardly. The liquid mixture retracted into the volume changer 441 is diluted to a degree so as not to gelatinize with the first liquid L1 in the first passage 44 (volume changer 441) while the liquid mixture retracted into the volume changer 451 is diluted to a degree so as not to gelatinize with the second liquid L2 of the second passage 45 (volume changer 451). This makes it possible to positively inhibit or prevent the liquid mixture remaining in the sprayer 1E from solidifying. Accordingly, the sprayer 1E can be used again in applying the mixed liquid to a diseased region.

In the sprayer 1E, positively drawing the liquid mixture into the volume changer 441, as well as the volume changer 451, requires a proper setting of the change amount (volume) that increases upon changing over from contraction to expansion of the volume changer 441.

The volume changer 441 and the volume changer 451 are arranged in different positions with respect to the lengthwise extent of the nozzle 4. That is, the two volume changers 441, 451 are longitudinally (axially) shifted or offset relative to one another. This arrangement helps prevent interference between the expanded volume changer 441 and the expanded volume changer 451. In the illustrated embodiment shown in Figs. 19-20, the volume changer 441 is positioned forwardly of the other volume changer 451.

The material forming the balloons constituting the volume changers 441, 451 is not especially limited. Examples of materials which can be used include an elastic material, e.g. rubber material such as natural rubber or butyl rubber, a thermoplastic elastomer based on polyurethane, polyester, polyamido, orefin or styrene, or a mixture thereof.

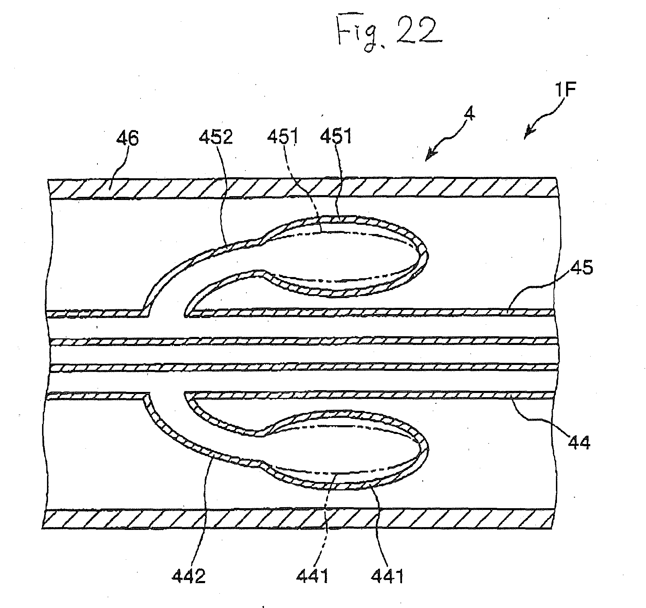

Fig. 22 illustrates a portion of a sprayer according to a seventh embodiment, at a position close to the volume changer. The description below primarily discusses aspects of the sprayer that differ from the embodiments described above. Features in this embodiment that are the same as those associated with the embodiments described above are designated by the same reference numerals and a detailed discussion of such features is not repeated. For the sake of explanatory convenience, the left and right sides in Fig. 22 are respectively referred to as the "front" and "rear (base)" sides.

This seventh embodiment is similar to the sixth embodiment except that the volume changers are differently positioned and slightly differently configured.

In the sprayer 1F shown in Fig. 22, one volume changer 441 is at an end of a branch 442 extending from and fluidly connected to the first passage 44, while the other volume changer 451 is at the end of a branch 452 extending from and connected to the second passage 45. The volume changers 441, 451 are each constructed as a balloon that expands and contracts depending upon the pressure in the third passage 46 (the internal pressure change of the gas G in the third passage 46) . The area indicated by the two-dot chain line in Fig. 22 shows the contracted state of the balloon.

The capacity or volume of each of the volume changers or volume changing portions 441, 451 in the state in which the liquid mixture ceases from being ejected (see the area indicated by the solid line in Fig. 22), is greater than that in the state in which the liquid mixture is being ejected (see the area indicated by the two-dot chain line in Fig. 22). This separates the liquid mixture into a portion drawn into the first passage 44 and a portion drawn into the second passage 45, whereby the liquid mixture is diluted in those passages in a manner similar to the sixth embodiment. This can positively prevent the liquid mixture remaining in the sprayer 1F from gelatinizing. Accordingly, the sprayer 1F can be used again in applying the liquid mixture to a diseased region.

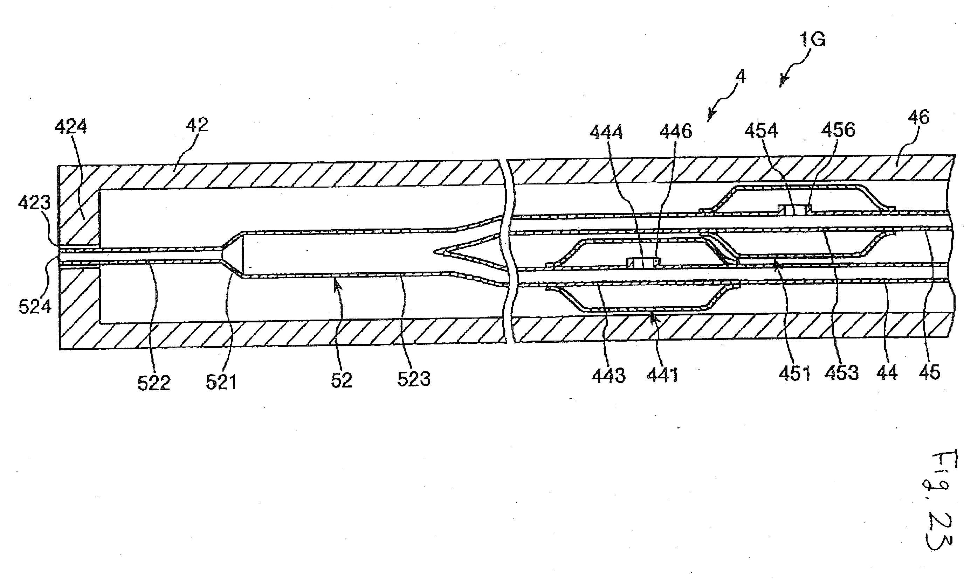

Figs. 23-25 show the nozzle of a sprayer according to an eighth embodiment. The following description addresses aspects of the nozzle or sprayer that differ from the embodiments described above. Features in this embodiment that are the same as those associated with the embodiments described above are designated by the same reference numerals and a detailed discussion of such features is not repeated. For the sake of explanatory convenience, the left and right sides in Figs. 23-25 are referred to as the "front" and "rear (base)" sides.

The present embodiment is similar to the sixth embodiment except that the sprayer in this eighth embodiment has passage-securing means.

In the sprayer 1G shown in Figs. 23-25, a rigid tube 443 passes through the volume changer or volume changing portion 441, and a rigid tube 453 passes through the volume changer or volume changing portion 451.

The rigid tube 443 is provided with one side hole 444 forming a circular opening in its tube wall. In the illustrated embodiment, the side hole 444 is positioned in the lengthwise center of the volume changer 441. The interior of the rigid tube 443 and the interior of the volume changer 441 are in communication with each other through the side hole 444.

The rigid tube 453 is also provided with one side hole 454 forming a circular opening in its tube wall. In the illustrated embodiment, the side hole 454 is positioned in the lengthwise center of the volume changer 451. The interior of the rigid tube 443 and the interior of the volume changer 451 are in communication with each other through the side hole 454.

The rigid tube 443 and the rigid tube 453 are similar in structure and function, and so the following description pertaining to the rigid tube 443 is also applicable to the rigid tube 453.

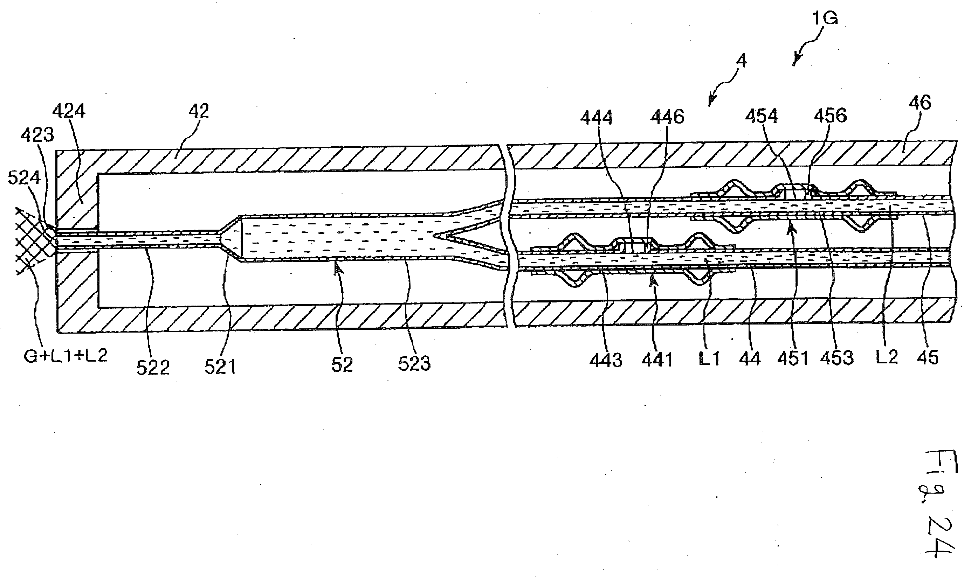

When the liquid mixture of the first and second liquids L1, L2 is ejected by operating the manipulator 8 in the state shown in Fig. 23, the volume changer 441 is compressed (the capacity or volume of the volume changer 441 decreases) and contracted, in accordance with the description set forth above in connection with other embodiments. At this time, the rigid tube 443 is pressed at its outer peripheral surface by the inner peripheral surface of the volume changer 441. The rigid tube 443 prevents or restrains the volume changer 441 from contracting further as generally shown in Fig. 24. This positively secures the passage of the first liquid L1 through the volume changer 441 (rigid tube 443). Accordingly, the first liquid L1 flows smoothly toward the liquid-mixture orifice 524 (confluence 52) without any restriction associated with contraction of the volume changer 441. In this manner, the rigid tube 443 serves as a passage-securing means that secures the passage of the first liquid L1 through the volume changer 441 even when the volume changer 441 is reduced in volume.

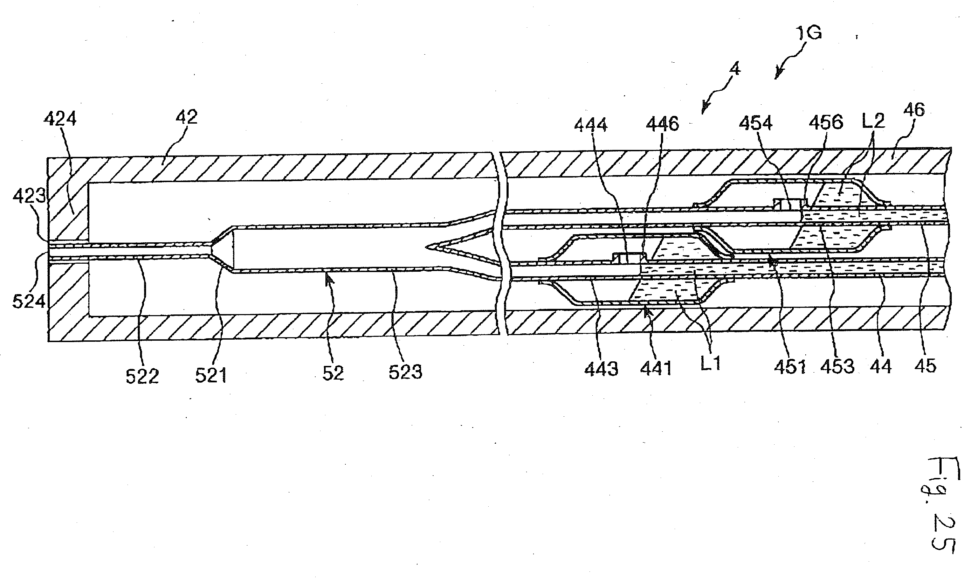

As mentioned before, after the application of the liquid mixture, the liquid mixture is retracted or drawn toward the rear rather than toward the confluence 52, i.e. into the volume changer 441 (rigid tube 443) as generally shown in Fig. 25. The liquid mixture retracted into the volume changer 441 is diluted to a degree so as not to gelatinize with the first liquid L1 existing in the first passage 44 (volume changer 441). Because the liquid mixture is retracted and received in the volume changer 441 (rigid tube 443), the liquid mixture is prevented from flowing in the reverse direction and leaking out through the liquid-mixture orifice 524.

The rigid tube 443 (as well as the rigid tube 453) is rigid to such an extent that the inner diameter of the rigid tube 443 does not substantially decrease even if the volume changer 441 is compressed and an inwardly directed pressure is applied to the outer peripheral surface of the rigid tube 443. The rigid tube 443 is thus able to serve its purpose and function as a passage-securing means. Here, the description that the inner diameter of the rigid tube 443 "does not substantially decrease" refers to an inner diameter reduction of 10% or smaller.

The material forming the rigid tube 443 to impart rigidity characteristics such as discussed above is not limited to a particular material. The material may be a metal material such as stainless steel, aluminum or aluminum alloy, rigid plastic in various type, or the like.

As shown in Figs. 23-25, a ring-shaped (annular) lip (rib) 446 is formed around the edge of the side hole 444 of the rigid tube 443, protruding from the outer peripheral surface of the rigid tube 443. Similarly, a ring-shaped (annular) lip (rib) 456 is also formed around the edge of the side hole 454 of the rigid tube 453, protruding from the outer peripheral surface of the rigid tube 453.

The lip 446 is advantageous in that it helps the inner surface of the volume changer 441 from closely contacting the portion of the outer periphery of the rigid tube 44 in the neighborhood of the side hole 444 when the volume changer 441 contracts as shown in Fig. 24. The same also applies to the other annular lip 456. The volume changer 441 thus smoothly deforms (changes) from a contracted state to an expanded state. In this manner, the lip 446 (and also the lip 456) serves as a close-contact preventing means that prevents at least a part of the inner peripheral surface of the volume changer 441 from contacting the outer peripheral surface of the rigid tube 443 when the volume changer 441 contracts.

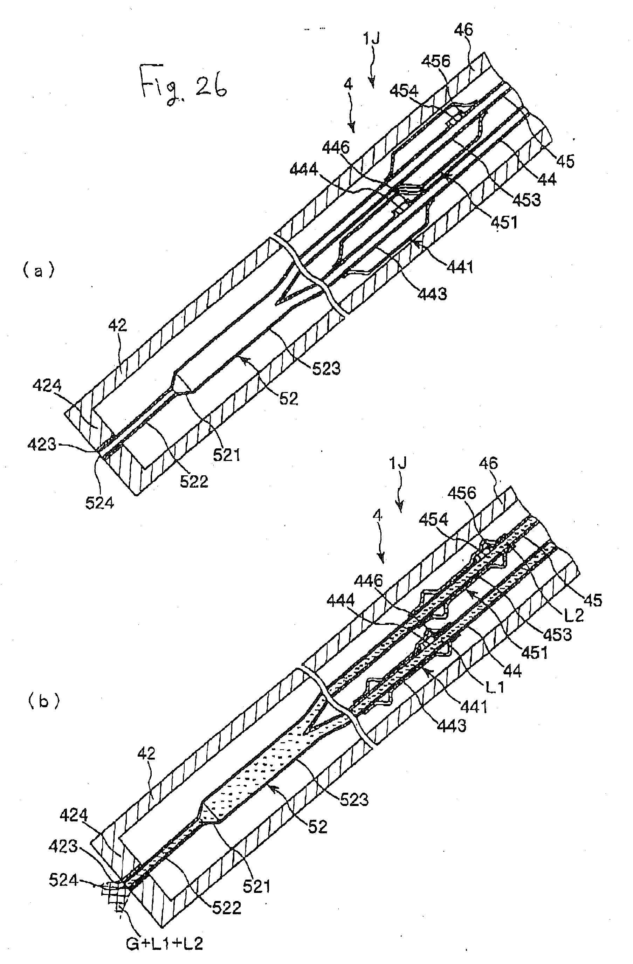

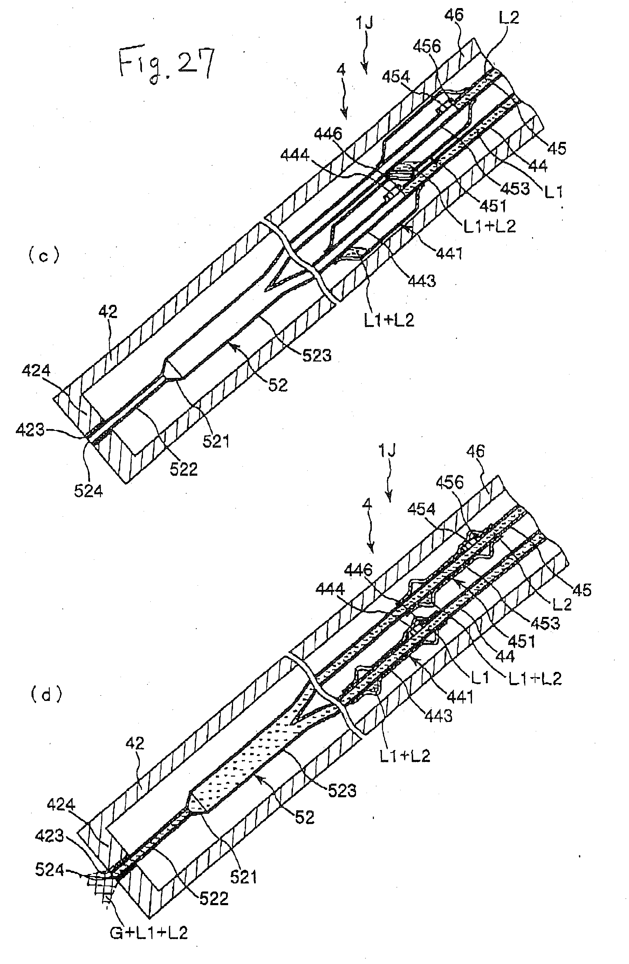

Figs. 26 and 27 illustrate a portion of the nozzle of a sprayer according to a ninth embodiment, illustrating chronologically a change in application state. The description below addresses aspects of the sprayer that differ from the embodiments described above. Features in this embodiment that are the same as those associated with the embodiments described above are designated by the same reference numerals and a detailed discussion of such features is not repeated. For the sake of explanatory convenience, the lower left side in Figs. 28 and 29 is referred to as the "front" side, the upper right side is referred to as the "rear (base) " side, the upper side is referred to as the "upper" side or "above", and the lower side is referred to as the "lower" side or "below".

This ninth embodiment is similar to the eighth embodiment except that the position of the side hole in the rigid tubes is different. The rigid tubes 443, 453 in this embodiment are the same as one another in terms of structure and function, and so the description which follows pertaining to the rigid tube 443 applies equally to the other rigid tube 453.

In the rigid tube 443 of the sprayer 1J shown in Figs. 26 and 27, the side hole 444 is arranged at a position closer to the base end of the volume changer or volume changing portion 441, i.e. on the upstream side (closer to the base or rear end) of the positioning of the side hole 444 of the rigid tube 443 in the eighth embodiment.

During use of the sprayer 1J, the front end of the nozzle 4 (nozzle head 42) is typically directed downward in the manner shown in Fig. 26A. This is also the same with the other embodiments of the sprayers 1-li. In this state, the manipulator 8 is operated to eject the liquid mixture (liquid mixture of the first and second liquids L1, L2) as generally shown in Fig. 26B. When the ejection of the liquid mixture ceases, the liquid mixture is retracted toward the rear rather than being urged toward the confluence 52, as noted before. The retracted liquid mixture includes a portion (L1 + L2) entering from the rigid tube 443 into the volume changer 441 through the side hole 444 and a portion staying in the first passage 44 (rigid tube 443) as shown generally in Fig. 27(a). The liquid mixture in the first passage 44 is diluted to a degree so as not to gelatinize with the first liquid L1 in the first passage 44. Meanwhile, the liquid mixture (L1 + L2) entering the volume changer 441 is at a location below the side hole 444, i.e. distant from the side hole 444.

When the sprayer 1J is used again, it is used with the front end of nozzle 4 directed downwardly in a manner similar to that just described such as generally shown in Fig. 27 (d). In this case, because the liquid mixture entering volume changer 441 is located fully above the side hole 444, the relevant liquid mixture is positively prevented from flowing reverse into rigid tube 443. This can positively prevent the liquid mixture received in the volume changer 441 from ejecting.

As shown in Figs. 26 and 27, the side hole 444 (as well as the side hole 454) is preferably formed in such a position that it opens upward (toward above) when the sprayer 1J is gripped in use. This makes it possible to provide side hole 444 as distant as possible from the liquid mixture entering volume changer 441. Therefore, when the sprayer 1J is used again, the liquid mixture received in the volume changer 441 is positively prevented from ejecting.

Although the sprayer described here has been explained so far by way of illustrated embodiments, the invention is not limited to those embodiments. Each of the features constituting the sprayer can be replaced with other features or structures that are capable of performing the same or similar functions. Also, additional features can be added.

The sprayer can also embody a combination of two or more structures (features) of the disclosed embodiments.

The confluence described in connection with the sixth embodiment may be provided in, for example, the first and third to fifth embodiments.

In the first to fifth embodiments, the volume changer may be constructed with a balloon as the volume changer similar to the sixth embodiment.

In the first to fifth embodiments, the volume changer may be replaced with a volume changer provided through a branch, as in the seventh embodiment.

The inner surface of the passage is preferably hydrophobic in a portion closer to the front end than the volume changer. By virtue of the hydrophobic nature, liquid is repelled upon the passage, thus being suppressed or prevented from staying in the passage. In order to provide the inner surface with hydrophobic properties, the inner surface can be provided with, for example, a material of fluorinated resin such as polytetrafluoro-ethylene or an olefin resin such as polypropylene or polyethylene.

The principles, embodiments and modes of operation of the sprayer have been described in the foregoing specification, but the invention which is intended to be protected is not to be construed as limited to the particular embodiments disclosed. The embodiments described herein are to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

## Claims

1. A method of delivering a liquid mixture to a region of a body, the method comprising:
ejecting a liquid mixture towards a region of a body by conveying first and second liquids in a forward direction along first and second liquid transfer passages respectively, mixing the liquids to produce the liquid mixture, and directing the liquid mixture at the body region;
ceasing ejecting the liquid mixture at the body region;
retracting the first liquid at a forward portion of the first liquid transfer passage in a rearward direction opposite the forward direction.

2. The method of Claim 1, wherein the liquid mixture is ejected by operating a presser, and wherein the first liquid at the forward portion of the first liquid transfer passage is retracted when operation of the presser ceases.

3. The method of Claim 1, further comprising compressing a volume changing portion fluidly connected to the first liquid transfer passage as the liquid mixture is being ejected, and allowing the volume changing portion to expand when the ejection of the liquid mixture ceases to retract the first liquid at the forward portion of the first liquid transfer passage in the rearward direction.

4. The method of Claim 3, wherein the first and second liquids are mixed in a confluence portion to form the liquid mixture, and wherein the ejection of the liquid mixture comprises ejecting the liquid mixture in the confluence portion through a nozzle.

5. The method of Claim 3, wherein the first and second liquids are conveyed to a nozzle head and are ejected through respective nozzles in the nozzle head, the first and second liquids being mixed after ejection from the respective nozzles to produce the liquid mixture.

6. The method of Claim 3, wherein the volume changing portion is compressed by supplying a gas to an interior of a chamber in which is located the volume changing portion, the gas supplied to the chamber applying a compression force to the volume changing portion which compresses the volume changing portion, the supply of the gas to the chamber ceasing when ejection of the liquid mixture ceases, and the volume changing portion expanding after the supply of the gas to the chamber ceases.

7. The method of Claim 3, wherein the volume changing portion is compressed by supplying a gas to an inflatable member positioned adjacent the volume changing portion to inflate the inflatable member, the inflation of the inflatable member applying a compression force to the volume changing portion which compresses the volume changing portion, the supply of the gas to the inflatable member ceasing when ejection of the liquid mixture ceases to thereby deflate the inflatable member, and the volume changing portion expanding as the inflatable member is deflated.

8. A sprayer comprising:
a supplier that supplies first and second liquids possessing different compositions;
two liquid transfer lines connected to the supplier and through which a respective one of the first and second liquids is to be supplied from the supplier;
at least one orifice in fluid communication with the two liquid transfer lines;
a volume changing portion on at least one of the two liquid transfer lines that is deformable to change an internal capacity of the volume changing portion;
a deformer that deforms the volume changing portion; and
the deformer being operable to deform the volume changing portion, whereupon a volume of the volume changing portion increases when ejection of the liquid ceases as compared to when the liquid is being ejected.

9. The sprayer of Claim 8, wherein the liquid in the liquid transfer line, on which the volume changer is provided, is retracted rearwardly away from the orifice when the volume of the volume changing portion increases.

10. The sprayer of Claim 8, wherein the liquid transfer lines each include a passage-forming portion through which the liquid passes, the passage-forming portion of the at least one of the two liquid transfer lines constituting the volume changing portion.

11. The sprayer of Claim 8, wherein the volume changing portion branches from the at least one of the two liquid transfer lines through a branch.

12. The sprayer of Claim 11, wherein the volume changing portion comprises an expandable and contractable balloon that expands and contracts depending upon operation of the deformer.

13. The sprayer of Claim 8, wherein the deformer comprises an inflatable member connected to a gas source by a gas passage, the inflatable member being positioned adjacent the volume changing portion and being inflatable upon supply of the gas from the gas source to compress and reduce the volume of the volume changing portion.

14. The sprayer of Claim 13, wherein the at least one orifice is a first orifice provided in a nozzle head, the nozzle head being provided with another orifice connected to the gas passage, the another orifice being concentrically positioned relative to the first orifice.

15. The sprayer of Claim 8, wherein each of the two liquid transfer lines is provided with one of the volume changing portions, the deformer comprising an inflatable member connected to a gas source by a gas passage, the inflatable member being positioned adjacent both of the volume changing portions and being inflatable upon supply of the gas from the gas source to compress and reduce the volume of both of the volume changing portions.

16. The sprayer of Claim 15, wherein the at least one orifice is a pair of liquid orifices each connected to one of the liquid transfer lines and provided in a nozzle head, the gas passage being fluidly communicated with a pair of gas nozzles provided in the nozzle head, each of the gas nozzles being concentric with one of the liquid nozzles.

17. The sprayer of Claim 8, wherein the deformer comprises a gas source, the gas source being connected to a gas passage, the volume changing portion being positioned in the gas passage and being compressed when gas is supplied from the gas source to the gas passage.

18. The sprayer of Claim 17, wherein the orifice is a liquid orifice provided in a nozzle head, the gas passage being connected to a gas nozzle in the nozzle head that is concentric to the liquid nozzle.

19. The sprayer of Claim 8, wherein each of the liquid transfer lines is provided with a respective volume changing portion, the deformer comprising a gas source, the gas source being connected to a gas passage, each of the volume changing portions being positioned in the gas passage and being compressed when gas is supplied from the gas source to the gas passage.

20. The sprayer of Claim 19, wherein the orifice is a liquid orifice provided in a nozzle head, the gas passage being connected to a gas nozzle in the nozzle head that is concentric to the liquid nozzle.

21. The sprayer of Claim 8, wherein forward ends of the liquid transfer lines are connected to one another at a confluence portion at which the first and second liquids are mixed with one another, the confluence portion being connected to the orifice.

22. The sprayer of Claim 8, wherein the at least one of the two liquid transfer lines is rigid and is provided with a hole communicating an interior of the at least one liquid transfer line to the volume changing portion.

23. The sprayer of Claim 8, wherein the deformer comprises a gas source which supplies gas to a region adjacent the volume changing portion to apply a compressive force to the volume changing portion which reduced the volume of the volume changing portion, and wherein a portion of the first liquid transfer passage constitutes the volume changing portion, and further comprising a spacer positioned inside the first liquid transfer passage to prevent the volume changing portion of the first liquid transfer from being completely closed when the gas is supplied to the region adjacent the volume changing potion.
